(19) Europäisches Patentamt / European Patent Office / Office européen des brevets

(11) **EP 2 951 722 B1**

(12) **EUROPEAN PATENT SPECIFICATION**

(45) Date of publication and mention
of the grant of the patent:
**16.05.2018 Bulletin 2018/20**

(21) Application number: **14701802.2**

(22) Date of filing: **31.01.2014**

(51) Int Cl.:
*G06F 17/18* (2006.01)       *G01N 15/14* (2006.01)
*G01N 33/569* (2006.01)     *G06F 19/24* (2011.01)

(86) International application number:
**PCT/EP2014/051963**

(87) International publication number:
**WO 2014/118343 (07.08.2014 Gazette 2014/32)**

(54) **PROCESS FOR IDENTIFYING RARE EVENTS**

PROZESS ZUR IDENTIFIKATION VON SELTENEN EREIGNISSEN

PROCÉDÉ D'IDENTIFICATION D'ÉVÉNEMENTS RARES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **31.01.2013 EP 13153512**

(43) Date of publication of application:
**09.12.2015 Bulletin 2015/50**

(73) Proprietors:
- **UNIVERSITE DE MONTPELLIER
  34090 Montpellier (FR)**
- **Centre Hospitalier Universitaire de Montpellier
  34295 Montpellier Cedex 05 (FR)**
- **INRIA - Institut National de Recherche en
  Informatique et en Automatique
  78153 Le Chesnay Cedex (FR)**
- **Institut National de Recherche en Sciences et
  Technologies pour l'Environnement et
  l'Agriculture
  (IRSTEA)
  92761 Antony Cedex (FR)**

(72) Inventors:
- **CEZAR, Renaud
  F-30870 Clarensac (FR)**
- **IENCO, Dino
  F-34093 Montpellier Cedex 5 (FR)**
- **MAS, André
  F-34095 Montpellier Cedex 5 (FR)**
- **MASSEGLIA, Florent
  F-34990 Juvignac (FR)**
- **PONCELET, Pascal
  F-34830 Jacou (FR)**

- **PUDLO, Pierre
  F-34090 Montpellier (FR)**
- **SZEKELY, Eniko
  New York, New York 10003 (US)**
- **TEISSEIRE, Maguelonne
  F-34093 Montpellier Cedex 5 (FR)**
- **VENDRELL, Jean-Pierre
  F-34170 Castelnau-le-Lez (FR)**

(74) Representative: **Monni, Richard
Cabinet LLR
11 boulevard de Sébastopol
75001 Paris (FR)**

(56) References cited:
- Y. GE ET AL: "flowPeaks: a fast unsupervised clustering for flow cytometry data via K-means and density peak finding", BIOINFORMATICS, vol. 28, no. 15, 1 August 2012 (2012-08-01), pages 2052-2058, XP055073112, ISSN: 1367-4803, DOI: 10.1093/bioinformatics/bts300
- DASH M ET AL: "'1+1>2': merging distance and density based clustering", DATABASE SYSTEMS FOR ADVANCED APPLICATIONS, 2001. PROCEEDINGS. SEVENTH INTERNATIONAL CONFERENCE ON APRIL 18-21, 2001, PISCATAWAY, NJ, USA,IEEE, 21 April 2001 (2001-04-21), pages 32-39, XP031977600, DOI: 10.1109/DASFAA.2001.916361 ISBN: 978-0-7695-0996-9

- K. MUMTAZ ET AL: "A Novel Density based improved k-means Clustering Algorithm - Dbkmeans", (IJCSE) INTERNATIONAL JOURNAL ON COMPUTER SCIENCE AND ENGINEERING, vol. 02, no. 02, 1 March 2010 (2010-03-01) , pages 213-218, XP055074829, ISSN: 0975-3397

- GUNJAN GUPTA ET AL: "Bregman bubble clustering", ACM TRANSACTIONS ON KNOWLEDGE DISCOVERY FROM DATA, vol. 2, no. 2, 1 July 2008 (2008-07-01), pages 1-49, XP055074826, ISSN: 1556-4681, DOI: 10.1145/1376815.1376817

**Description**

The present invention relates to a process for identifying rare events One method for characterizing heterogeneous cell populations is by flow cytometry. Using this technology, cells are labeled with antibodies conjugated to dyes. Flow cytometry can routinely detect 1, 2 or more immunofluorescent markers simultaneously in a quantitative manner. By combining multiple immunofluorescent labels with the light scattering properties of the cells it is possible to distinguish not only between cells of different lineages but between cells at various stages of maturation within those lineages. Populations identified by the flow cytometer can then be isolated using the cell sorting electronics available on the instrument.

he international application WO2006089190 discloses a method for detecting abnormal cells using a multidimensional analysis. This application discloses a method wherein cells of a determined sample are clustered and compared with a reference sample in order to identify abnormal cells, i.e. rare cells that are only present in said determined sample.

This method allows the identification of rare cells belonging to a large population, but requires the use of a normal population of cells.

The required comparison step can be difficult to carry out when it is not possible, for an individual or for a population, to obtain said control sample.

Therefore, there is a need to provide a method allowing the identification of a subpopulation of cells within a large population of cells, whatsoever the sample, and which is not dependent upon a reference sample.

The aim of the invention is to overcome the above inconvenient.

Another aim of the invention is to provide a method allowing the detection of rare cells in a direct, simple, and reproducible manner, independently of an engineer intervention.

Still another aim of the invention is to provide a computer program able to carry out the above method.

Y. Ge et al: "flowPeaks: a fast unsupervised clustering for flow cytometry data via K-means and density peak finding", BIOINFORMATICS, vol. 28, no. 15, August 2012, pages 2052-2058, discloses clustering and outlier detection for flow cytometry using a two-step approach. First, a high number of clusters is determined using K-means. Then, a Gaussian finite mixture model is used to merge clusters by considering peak distances. A density measure is used to detect outliers.

The present invention relates to a method for identifying a subpopulation of specific cells among a large population of cells, as set forth in claim 1. The present invention is based on the unexpected observation made by the inventors that applying a multiple data analysis in a large population of cells allows the identification of small populations of cells belonging within said large population. The method according to the invention comprises

- a first step of labeling cells, and identifying the labeling,
- a second step of clustering cells; and adjusting the clusterisation step, and
- a step of adjusting the clusterisation, in order to obtain the most precise cluster containing most of the cells of the population, sad clustered being eliminated in order to reveal rare cells.

First step : labeling cells and detecting the labeled cells.

In order to carry out the process according to the invention, all the cells belonging to the analyzed population are labeled with n- reagents, n being equal to or greater than 2.

All the cells belonging to the large population of cells are labeled with n-reagents, preferably n-different reagents each of them reacting with n different components of each cells belonging to said population.

In the invention, $n \geq 2$, which means that n equals to 2, 3, 4, 5, 6, 7, 8, 9, 10, 11, 12, 13, 14, 15, 16, 17, 18, 19, 20 or more. The number of reagent should be greater than 1 in order to discriminate cells, and the number of reagent would only depend upon the ability to the practitioner to detect them simultaneously or sequentially.

The n- reagents are specific to components of each cells, their interaction with said component defining the presence, the absence or the amount of said component in each cells.

The n- reagents used to label the cells of the large population can be detected. The detection can be carried out by specific means depending upon their nature or their physical or chemical or both properties. For instance, and without limiting the scope of the invention, the reagents can be fluorescent, magnetics, phosphorescent, radioactive, water insoluble, activable, inducible...

Commonly, the n- reagents are antibodies that specifically recognize one specific component of cells. To be detectable, the antibodies are coupled with detectable compounds, such as fluorescent dye, beads, in particular magnetic bead, enzymes ...

The reagents can also be intercalating agents of DNA or any other molecules.

The reagent that have reacted with cells of the population are detected such that each cell is identified by n-specific detections, which allow to assign each cells in a specific position in the n-dimension space. The presence, absence or amount determines the coordinates in a determined n- dimension.

Second step : clusterisation and grouping.

When each cell is assigned to a specific position within said n- dimension space, a clusterisation step is carried out.

Cluster analysis or clustering is the task of grouping a set of objects in such a way that objects in the same group (called cluster) are more similar (in some sense or another) to each other than to those in other groups (clusters). It is a main task of explorative data mining, and a common technique for statistical data analysis used in many fields.

Cluster analysis itself is not one specific algorithm, but the general task to be solved.

It can be achieved by various algorithms that differ significantly in their notion of what constitutes a cluster and how to efficiently find them. Popular notions of clusters include groups with :

- low distances among the cluster members,
- dense areas of the data space,
- intervals or particular statistical distributions.

Clustering can therefore be formulated as a multi-objective optimization problem. The appropriate clustering algorithm and parameter settings (including values such as the distance function to use, a density threshold or the number of expected clusters) depend on the individual data set and intended use of the results. Cluster analysis as such is not an automatic task, but an iterative process of knowledge discovery or interactive multi-objective optimization that involves trial and failure. It will often be necessary to modify preprocessing and parameters until the result achieves the desired properties.

The notion of a "cluster" cannot be precisely defined, which is one of the reasons why there are so many clustering algorithms. There of course is a common denominator: a group of data objects. However, different cluster models are used, and for each of these cluster models again different algorithms can be given. The notion of a cluster found by different algorithms vary significantly in their properties, and understanding these "cluster models" is key to understanding the differences between the various algorithms. Typical cluster models include:

- Connectivity models: for example hierarchical clustering builds models based on distance connectivity.
- Centroid models: for example the k-means algorithm represents each cluster by a single mean vector.
- Distribution models: clusters are modeled using statistical distributions, such as multivariate normal distributions used by the Expectation-maximization algorithm.
- Density models: for example DBSCAN and OPTICS defines clusters as connected dense regions in the data space.
- Subspace models: in Biclustering (also known as Co-clustering or two-mode-clustering), clusters are modeled with both cluster members and relevant attributes.
- Group models: some algorithms (unfortunately) do not provide a refined model for their results and just provide the grouping information.
- Graph-based models: a clique, i.e., a subset of nodes in a graph such that every two nodes in the subset are connected by an edge can be considered as a prototypical form of cluster. Relaxations of the complete connectivity requirement (a fraction of the edges can be missing) are known as quasi-cliques.

In centroid-based clustering, clusters are represented by a central vector, which may not necessarily be a member of the data set. When the number of clusters is fixed to k, k-means clustering gives a formal definition as an optimization problem: find the $k$ cluster centers and assign the objects to the nearest cluster center, such that the squared distances from the cluster are minimized.

The optimization problem itself is known to be NP-hard, and thus the common approach is to search only for approximate solutions. A particularly well known approximative method is Lloyd's algorithm, often actually referred to as "k-means algorithm". It does however only find a local optimum, and is commonly run multiple times with different random initializations. Variations of k-means often include such optimizations as choosing the best of multiple runs, but also restricting the centroids to members of the data set (k-medoids), choosing medians (k-medians clustering), choosing the initial centers less randomly (K-means++) or allowing a fuzzy cluster assignment (Fuzzy c-means).

Most k-means-type algorithms require the number of clusters - k - to be specified in advance, which is considered to be one of the biggest drawbacks of these algorithms. Furthermore, the algorithms prefer clusters of approximately similar size, as they will always assign an object to the nearest centroid. This often leads to incorrectly cut borders in between of clusters (which is not surprising, as the algorithm optimized cluster centers, not cluster borders).

K-means has a number of interesting theoretical properties. On one hand, it partitions the data space into a structure known as Voronoi diagram. On the other hand, it is conceptually close to nearest neighbor classification and as such popular in machine learning. Third, it can be seen as a variation of model based classification, and Lloyd's algorithm as a variation of the Expectation-maximization algorithm for this model discussed below.

Advantageously, when using k-means in the invention, each of the k- clusters are defined by

- their centroid $C_k$ and
- their radius D.

In other words, for k=3, cluster 1 is defined by $C_1$ and D, cluster 2 is defined by $C_2$ and D and cluster 3 is defined by $C_3$ and D. All the clusters have the same radius, but have a different centroid.

The centroid, also called geometric center, or barycenter, of a plane figure or two-dimensional shape X is the intersection of all straight lines that divide X into two parts of equal moment about the line. Informally, it is the "average" (arithmetic mean) of all points of X.

The definition extends to any object X in n-dimensional space: its centroid is the intersection of all hyperplanes that divide X into two parts of equal moment.

The inventors advantageously used DenseKMeans, a modified variant of k-means, designed to find and cluster only points that lie in dense regions of the space.

The skilled person, in view of the above definition is able to obtain clusters according to the invention.

When clusters are determined, the adjacent clusters are grouped to obtain larger clusters.

In the invention, adjacent clusters are such that the Euclidian distance between the centers Ck of two clusters is lower than twice the radius DMAX. In mathematics, the Euclidean distance between the points p and q is the length of the line segment connecting them. In general, for an n-dimensional space, the distance is

$$\mathbf{d(p,q)} = \sqrt{(\mathbf{p_1} - \mathbf{q_{1)}})^2 + (\mathbf{p_2} - \mathbf{q_2})^2 + \cdots + (\mathbf{p_n} - \mathbf{q_n})^2}.$$

When adjacent clusters are identified, and larger clusters determined, the center of said larger clusters $C_{lk}$ are estimated. Moreover, the covariance matrix is also estimated.

A covariance matrix (also known as dispersion matrix or variance covariance matrix) is a matrix whose element in the i, j position is the covariance between the $i^{th}$ and $j^{th}$ elements of a random vector (that is, of a vector of random variables). Each element of the vector is a scalar random variable, either with a finite number of observed empirical values or with a finite or infinite number of potential values specified by a theoretical joint probability distribution of all the random variables.

In the invention, each of the larger clusters is thus defined by their newly defined center and the covariance matrix.

Third step: delimiting the cluster and eliminating the dense cluster.

Further to the determination of the larger clusters, a sliding region is defined, for each cluster by increasing the radius size, in each of the n- dimension, by a factor $\varepsilon$. The factor $\varepsilon$ varies from 0.01 to 1, advantageously from 0.05 to 0.5, in particular $\varepsilon$ is 0.1.

Then, the Mahalanobis distance for each cell that belongs to said sliding region is calculated.

The Mahalanobis distance is the distance of a case from the centroid in the multidimensional space, defined by the correlated independent variables (if the independent variables are uncorrelated, it is the same as the simple Euclidean distance). Thus, this measure provides an indication of whether or not an observation is an outlier with respect to the independent variable values.

Mahalanobis distance between two samples (x,y) of a random variable is defined as

$$\mathbf{d(x,y)} = \sqrt{(x - y)^{\mathrm{T}} \Sigma^{-1} (x - y)}$$

wherein $\Sigma^{-1}$ is the inverse of the covariance matrix.

Within the sliding region, all the cells have a Mahalanobis distance lower than $D_{lk}(1+\varepsilon)$, wherein $D_{lk}$ is the radius of a larger cluster k.

The cells of interest belonging to the sliding region do not belong to the larger cluster.

This step intends to enhance gradually, by a factor $\varepsilon$, the size of the larger cluster, in order to obtain all the cells that belong to said larger cluster, but which are located to the border of said larger cluster.

When the sliding region is determined, the density of said sliding region is evaluated. If the density is higher than a value N, N being upper than 10, preferably N varies from 10 to 1000, in particular N varies from 10 to 500, (i.e. which means that the density of the sliding region is higher than a determined value N), the sliding region is considered to belong to the larger cluster.

Advantageously, the density N varies from 10 to 1000, in particular from 10 to 500, and in particular equals to 10.

Thus, steps e and f of the process according to the invention are repeated p times until the density of the sliding region p, determined by the cells having a Mahalanobis distance lower than $D_{lk}(1+\varepsilon)^p$, is lower than N. In this case, this means that the cells of the sliding region n does not belong to the larger clusters (which has been enlarged p times by the factor $\varepsilon$).

At this stage, all the cells belonging to the larger clusters are eliminated, and among the remaining cells are present the expected subpopulation of specific cells, i.e. rare cells.

Advantageously, the invention relates to the method as defined above, wherein said n- reagents are fluorescent reagents that interact with cellular proteins, lipids, glucids or nucleic acid molecules.

A fluorescent compound absorbs light energy over a range of wavelengths that is characteristic for that compound. This absorption of light causes an electron in the fluorescent compound to be raised to a higher energy level. The excited electron quickly decays to its ground state, emitting the excess energy as a photon of light. This transition of energy is called fluorescence.

The range over which a fluorescent compound can be excited is termed its absorption spectrum. As more energy is consumed in absorption transitions than is emitted in fluorescent transitions, emitted wavelengths will be longer than those absorbed. The range of emitted wavelengths for a particular compound is termed its emission spectrum.

As mentioned above, said n- reagents are advantageously fluorescent compounds or molecules that directly, or indirectly, interact with component of the cells, such as proteins, lipids, glucids or nucleic acid molecules. This also includes glycoproteins, phospholipids and modifies glucids.

Intercalating molecules of DNA can be used as fluorescent molecules interacting directly with DNA. Propidium iodide (PI) or 7-Aminoactinomycin D (7-AAD) are commonly used as fluorescent marker to label DNA. The skilled person can easily choose any other agent having the same properties.

Antibodies coupled, in particular in their Fc region, with fluorescent compounds can also be used to detect proteins, lipids, glucids, glycoforms of proteins and lipids. As fluorescent dyes, the following compounds are commonly used: FITC (Fluorescein Isothiocyanate), Alexa Fluor® 488, R-PE (R-Phycoerythrin), PE-Texas Red, PE-Alexa Fluor® 610, PE-Cy5, PerCP-Cy5.5, PerCP-eFluor® 710, PE-Cy7, Alexa Fluor® 532, APC (Allophycocyanin), eFluor® 660, Alexa Fluor® 647, Alexa Fluor® 700, APC-eFluor® 780, eFluor® 450, eFluor® 605NC, eFluor® 625NC, eFluor® 650NC, Pacific Blue®, Pacific Orange®, Brillant Violet™ 421, Brillant Violet™ 510, Brillant Violet™ 570, Brillant Violet™ 605, Brillant Violet™ 650, Brillant Violet™ 711 and Brillant Violet™ 785. This list is not limitative, and the skilled person can easily choose the most appropriate fluorescent dyes.

Advantageously, the n- reagents used in the process according to the invention specifically detect the presence, the absence or the amount of transmembrane proteins called cluster of differentiation markers (CD). It is to be notices that some dyes can be located into the cells (intracellular CD).

The cluster of differentiation antigens are membrane proteins mainly expressed on leukocytes. A small number are also expressed on endothelial cells, erythrocytes, and stem cells. Cluster of differentiation antigens are commonly used as cell markers, allowing cells to be defined based on what molecules are present on their surface. For example, two commonly-used CD molecules are CD4 and CD8, which are, in general, used as markers for two different subtypes of T-lymphocytes, helper and cytotoxic T cells, respectively. CD4 is specifically recognized and bound by HIV, leading to viral infection and destruction of CD4+ T cells. The relative abundance of CD4+ and CD8+ T cells is a gold marker used to monitor the progression of an HIV infection. Detection the expression of cluster of differentiation (CD) antigens is supposed to be developed as diagnosis methods in some diseases, such as cardiovascular disease, and tumors.

In human, most of the known CD markers are the following ones: CD1a, CD1b, CD1c, CD1d, CD1e, CD2, CD3δ, CD3ε, CD3γ, CD4, CD5, CD5L, CD6, CD7, CD8a, CD8b, CD9, CD10 / Neprilysin, CD11a, CD11b / Integrin alpha M, CD11c, Cdw12, CD13 / ANPEP, CD14, CD15, CD15s, CD15u, CD15su, CD16a / Fc gamma RIIIA, CD16b / Fc gamma RIIIB, CD16-2 / FCGR4, CD17, CD18 / Integrin beta 2 / TNFRSF3, CD19, CD20 / MS4A1, CD21, CD22, CD23 / FCER2, CD24, CD25 / IL-2R / IL-2RA, CD26 / DPP4, CD27 / TNFRSF7, CD27L / CD70 / TNFSF7, CD28, CD29, CD30 / TNFRSF8, CD30L / CD153 / TNFSF8, CD31 / PECAM1, CD32 /Fc gamma RII, CD32a / Fc gamma RIIA, CD32b(variant2), CD32b(variant3), CD33 / Siglec-3, CD34, CD34T, CD35, CD36 / SCARB3, CD36L1 / SCARB1, CD36L2 / LIMP-2 / SCARB2, CD37, CD38, CD39, CD40 / TNFRSF5, CD40L / CD154 / TNFSF5, CD41, CD42a, CD42b, CD42c / GP1BB, CD42d, CD43, CD44, CD44R, CD45, CD45RA, CD45RB, CD45RC, CD45RO, CD46, CD47, CD48 / SLAMF2, CD49a, CD49b, CD49c, CD49d / Integrin alpha 4, CD49e / Integrin alpha 5, CD49f, CD50 / ICAM-3, CD51 , CD52, CD53, CD54 / ICAM-1, CD55 / DAF, CD56/NCAM1, CD57, CD58, CD59, CD60a, CD60b, CD60c, CD61/Integrin beta 3, CD62E / E-Selectin, CD62L / L-Selectin, CD62P / P-Selectin, CD63, CD64 / Fc gamma RI, CD65, CD65s, CD66a / CEACAM1, CD66b / CD67 / CEACAM8, CD66c / CEACAM6, CD66d / CEACAM3, CD66e / CEACAM5, CD66f, CD68, CD69, CD70 / CD27L / TNFSF7, CD71 / TFRC, CD72, CD73 / NT5E, CD74, CD75 / ST6GAL1, CD75s, CD77, CD79a, CD79b, CD80 / B7-1, CD81, CD82 / KAI-1, CD83, CD84 / SLAMF5, CD85a, CD85b, CD85c, CD85d, CD85e, CD85f, CD85g, CD85h, CD85i, CD85j, CD85k, CD85I, CD85m, CD86 / B7-2, CD87 / PLAUR, CD88, CD89 / FCAR, CD90 / THY-1, CD91 / LRP1, CD92, CD93 / C1qR, CD94, CD95 / APO-1 / TNFRSF6, CD95L / CD178/TNFSF6, CD96, CD97, CD98 / SLC3A2, CD99, CD99L2, CD99R, CD100 / SEMA4D, CD101 CD102 / ICAM-2, CD103, CD104, CD105 / Endoglin, CD106 / VCAM-1, CD107a / LAMP-1, CD107b / LAMP2, Cdw108, CD109, CD110 / TPOR / C-MPL, CD111 / Nectin-1 / PVRL1, CD112 / Nectin-2, CD113 / Nectin-3, CD114 /G-CSFR, CD115 / CSF1R / MCSF Receptor, CD116 / GM-CSFR, CD117 / c-Kit, CD118 / LIFR, CD119 / IFNGR1, CD120a / TNFR1 / TNFRSF1A, CD120b / TNFR2 / TNFRSF1B, CD121a / IL-1R1, CD121b / IL-1R2, CD122 / IL-2RB, CD123 / IL-3RA, CD124 / IL-4R, CD125 / IL-5RA, CD126 / IL-6R, CD127 / IL-7RA, CD128 / CD181 / CXCR1, CD128b / CD182 / CXCR2, Cdw129, CD130 / gp130 / IL6ST, CD131 / IL-3RB / CSF2RB, CD132 / IL-2RG, CD133, CD134 / OX40 / TNFRSF4, CD135 / FLT3 / FLK2, CD136 / MST1R, CD137 / 4-1BB / TNFRSF9, CD137L / 4-1BBL / TNFSF9, CD138 / Syndecan-1 / SDC1, CD139, CD140a / PDGFRA, CD140b / PDGFRB, CD141, CD142 / Tissue Factor, CD143, CD144 / VE-Cadherin, CDw145, CD146 / MCAM, CD147 / EMMPRIN, CD148, CD150 / SLAM, CD151, CD152 / CTLA-4, CD153 / CD30L / TNFSF8, CD154 / CD40L / TNFSF5, CD155 / PVR, CD155b, CD156a / ADAM8, CD156b, CD156c, CD157 / BST1, CD158a, CD158b1, CD158b2 / KIR2DL3, CD158c, CD158d, CD158e1, CD158e2, CD158f, CD158g, CD158h, CD158i, CD158j, CD158k, CD158z, CD159a, CD159c, CD160, CD161, CD162 / PSGL-1, CD162R, CD163, CD164, CD165, CD166 / ALCAM, CD167a/DDR1/MCK10, CD168, CD169, CD170, CD171 / NCAM-L1, CD172a / SIRP alpha, CD172b / SIRP beta, CD172g / SIRP gamma, CD173, CD174, CD175, CD175s, CD176, CD177, CD178 / CD95L / TNFSF6, CD179a, CD179b, CD180 / RP105, CD181 / CD128 / CXCR1, CD182 / CD128b / CXCR2, CD183, CD184, CD185, CD186, CD191, CD192, CD193, CD194, CD195, CD196, CD197, CDw198, CDw199, CD200, CD200R, CD200R1, CD200R4, CD200RLa, CD201, CD202b / Tie2, CD203c, CD204 / MSR1, CD205, CD206, CD207 / Langerin, CD208 / DC-LAMP, CD209 / DC-SIGN, CD209b / SIGNR1, CD209g, CD210a / IL-10RA, CDw210b / IL-10RB, CD212 / IL12RB1, CD213a1 / IL-13RA1, CD213a2 / IL-13RA2, CD217 / IL-17R / IL-17RA, CD218a / IL-18R1 / IL-18RA, CD218b / IL-18RAP / IL-1R7, CD220 / Insulin R, CD221 / IGF1R, CD222, CD223, CD224, CD225, CD226 / DNAM-1, CD227 / MUC-1 / Mucin 1, CD228, CD229 / LY9, CD230, CD231, CD232, CD233, CD234, CD235a, CD235ab, CD235b, CD236, CD236R, CD238, CD239 / BCAM, CD240CE, CD240D, CD240DCE, CD241 CD242, CD243, CD244 / 2B4 / SLAMF4, CD245, CD246, CD247, CD248, CD249/ENPEP, CD252, CD253/TNFSF10/TRAIL, CD254/RANKL/OPGL/TNFSF11, CD255, CD256 / TNFSF13, CD257 / BLyS / TNFSF13B, CD258 / LIGHT / TNFSF14, CD261/ TRAIL R1 / TNFRSF10A, CD262 / TRAIL R2 / TNFRSF10B, CD263 / TRAIL R3 / TNFRSF10C, CD264 / TRAIL R4 / TNFRSF10D, CD265, CD266 / TWEAKR / TNFRSF12A, CD267 / TACI / TNFRSF13B, CD268 / BAFFR / TNFRSF13C, CD269 / TNFRSF17 / BCMA, CD270, CD271, CD272, CD273/B7-DC/PD-L2, CD274 / B7-H1 / PD-L1, CD275, CD276 / B7-H3, CD277, CD278 / ICOS / AILIM, CD279 / PD1 / PDCD1, CD280, CD281 / TLR1, CD282 / TLR2, CD283 / TLR3, CD284 / TLR4, CD286, CD288, CD289, CD290, CD292 / BMPR1A / ALK-3, CDw293 / BMPR1B / ALK-6, CD294, CD295 / LEPR, CD296, CD297, CD298, CD299 / DC-SIGNR, CD300a, CD300b, CD300c, CD300e, CD300f, CD301 / CLEC10A, CD302 / CLEC13A, CD303, CD304 / Neuropilin-1, CD305, CD306 / LAIR2, CD307a, CD307b, CD307c, CD307d, CD307e, CD309 / VEGFR2 / Flk-1, CD312, CD314 / NKG2D, CD315, CD316, CD316, CD317, CD318, CD319 / CRACC / SLAM7, CD320, CD321 / JAM-A / F11R, CD322 / JAM-B, CD324 / E-Cadherin / CDH1, CD325 / CDH2 / N-cadherin, CD326 / EpCAM, CD327, CD328, CD329, CD331 / FGFR1, CD332 / FGFR2, CD333 / FGFR3, CD334 / FGFR4, CD335, CD336 / NCR2 / NKp44, CD337 / NCR3 / Nkp30, CD338, CD339/JAG1/Jagged 1, CD340 / HER2 / ErbB2, CD344, CD349, CD350 / Frizzled-10 / FZD10, CD351, CD352, CD353, CD354, CD355, CD357, CD358, CD359, CD360, CD361, CD362 and CD363.

Combinations of specific markers are used to identify specific cell lines. For instance, the key markers (i.e. markers that are representative of a cell line) are:

- CD3, CD4 and CD8 for T cells,
- CD19 and CD20 for B cells,
- CD11c and CD123 for dendritic cells,
- CD56 for Natural Killer (NK) cells,
- CD34 for hematopoietic stem cells,
- CD14 and CD133 for monocytes/macrophages
- CD66b for granulocytes,
- CD41, CD 61 and CD62 for platelets,
- CD235a for erythrocytes,
- CD146 for endothelial cells, and
- CD326 for epithelial cells.

In one advantageous embodiment, the invention relates to the method previously defined, wherein the detecting step b. is carried out by flow cytometry.

Flow cytometry is a technology that simultaneously measures and then analyzes multiple physical characteristics of single particles, usually cells, as they flow in a fluid stream through a beam of light. The properties measured include a particle's relative size, relative granularity or internal complexity, and relative fluorescence intensity. These characteristics are determined using an optical-to-electronic coupling system that records how the cell or particle scatters incident laser light and emits fluorescence.

In the flow cytometer, particles are carried to the laser intercept in a fluid stream. Any suspended particle or cell from 0.2-150 micrometers in size is suitable for analysis.

Cells from solid tissue must be disaggregated before analysis. The portion of the fluid stream where particles are located is called the sample core. The scattered and fluorescent light is collected by appropriately positioned lenses. A combination of beam splitters and filters steers the scattered and fluorescent light to the appropriate detectors. The detectors produce electronic signals proportional to the optical signals striking them. List mode data are collected on each particle or event. The characteristics or parameters of each event are based on its light scattering and fluorescent properties. The data are collected and stored in the computer. This data can be analyzed to provide information about subpopulations within the sample through the laser intercept, they scatter laser light.

Light scattering occurs when a particle deflects incident laser light. The extent to which this occurs depends on the physical properties of a particle, namely its size and internal complexity. Factors that affect light scattering are the cell's membrane, nucleus, and any granular material inside the cell. Cell shape and surface topography also contribute to the total light scatter. Forward-scattered light (FSC) is proportional to cell-surface area or size. FSC is a measurement of mostly diffracted light and is detected just off the axis of the incident laser beam in the forward direction by a photodiode. FSC provides a suitable method of detecting particles greater than a given size independent of their fluorescence and is therefore often used in immunophenotyping to trigger signal processing.

Side-scattered light (SSC) is proportional to cell granularity or internal complexity. SSC is a measurement of mostly refracted and reflected light that occurs at any interface within the cell where there is a change in refractive index. SSC is collected at approximately 90 degrees to the laser beam by a collection lens and then redirected by a beam splitter to the appropriate detector.

More than one fluorochrome can be used simultaneously if each is excited at 488 nm and if the peak emission wavelengths are not extremely close to each other. The combination of FITC and phycoerythrin (PE) satisfies these criteria. It is to be noticed that a 488 mn laser can be used to detect 5 different fluorochromes (FITC, PE, ECD, PC-Cy5 or PC-Cy5.5 and PC-Cy7) and that a violet laser laser can be used to detect 7 different fluorochromes (for instance the above mentioned 7 Brillant Violet™ dyes) Although the absorption maximum of PE is not at 488 nm, the fluorochrome is excited enough at this wavelength to provide adequate fluorescence emission for detection. More important, the peak emission wavelength is 530 nm for FITC and 570 nm for PE. These peak emission wavelengths are far enough apart so that each signal can be detected by a separate detector. The amount of fluorescent signal detected is proportional to the number of fluorochrome molecules on the particle.

A flow cytometer has five main components:

- a flow cell - liquid stream (sheath fluid), which carries and aligns the cells so that they pass single file through the light beam for sensing
- a measuring system - commonly used are measurement of impedance (or conductivity) and optical systems - lamps (mercury, xenon); high-power water-cooled lasers (argon, krypton, dye laser); low-power air-cooled lasers (argon (488 nm), red-HeNe (633 nm), green-HeNe, HeCd (UV)); diode lasers (blue, green, red, violet) resulting in light signals
- a detector and Analogue-to-Digital Conversion (ADC) system - which generates FSC and SSC as well as fluorescence signals from light into electrical signals that can be processed by a computer
- an amplification system - linear or logarithmic
- a computer for analysis of the signals.

The process of collecting data from samples using the flow cytometer is termed 'acquisition'. Acquisition is mediated by a computer physically connected to the flow cytometer, and the software which handles the digital interface with the cytometer. The software is capable of adjusting parameters (i.e. voltage, compensation, etc.) for the sample being tested, and also assists in displaying initial sample information while acquiring sample data to insure that parameters are set correctly. Early flow cytometers were, in general, experimental devices, but technological advances have enabled widespread applications for use in a variety of both clinical and research purposes. Due to these developments, a considerable market for instrumentation, analysis software, as well as the reagents used in acquisition such as fluorescently-labeled antibodies has developed.

Modern instruments usually have multiple lasers and fluorescence detectors. The current record for a commercial instrument is four or five lasers and 18 fluorescence detectors. Increasing the number of lasers and detectors allows for multiple antibodies labeling, and can more precisely identify a target population by their phenotypic markers. Certain instruments can even take digital images of individual cells, allowing for the analysis of fluorescent signal location within or on the surface of cells. The invention relates to the method previously defined, wherein the clusterization step is achieved by carrying out a clustering algorithm which is a k-means derived algorithm. As mentioned above, the clustering algorithm is a modified k-means, algorithm in particular DenseKmeans.

DenseKmeans algorithm is explained in Example 1.

In an advantageous embodiment, the invention relates to the method previously defined, wherein said large population of cells form all animal or human fluids such as blood sample, cerebrospinal fluid, amniotic fluid, bronchoalveolar lavage fluid, breast milk and cervicovaginal liquids.

More advantageously, the invention relates to the method as defined above, for identifying a subpopulation of mature endothelial cells in a blood sample, wherein the cells of the large population are labeled with at least the following markers: CD45, CD105 and CD146.

More advantageously, the invention relates to the method as defined above, for identifying a subpopulation of progenitor endothelial cells in a blood sample, wherein the cells of the large population are labeled with at least the following markers: CD45, CD34, CD133, and CD309.

Advantageously, the invention relates to the method as defined above, for identifying a subpopulation of mature or progenitor endothelial cells in a blood sample, wherein the cells of the large population are labeled with at least the following markers: 7AAD, CD31, CD45, CD105, CD146, CD34, CD133, CD144 and CD309.

More advantageously, the invention relates to the method as defined above, for identifying a subpopulation of epithelial cells, wherein the cells of the large population are labeled with at least the following markers: CD326, CD45, antibodies directed to cytokeratins.

Advantageously, the invention relates to the method as defined above, for identifying a subpopulation of epithelial cells, wherein the cells of the large population are labeled with at least the following markers: CD44, CD326, CD45, antibodies directed to cytokeratins.

More advantageously, the invention relates to the method as defined above, for identifying a subpopulation of regulating B cells or of Epstein-Barr Virus (EBV) infected memory B cells, wherein the cells of the large population are labeled with at least the following markers: CD27, CD24, CD19, and IL-10 or the following markers: CD27, CD19 and antibodies directed to EBV antigens, respectively.

Advantageously, the invention relates to the method as defined above, for identifying a subpopulation of regulating B cells wherein the cells of the large population are labeled with at least the following markers: CD38, CD27, CD24, CD19, CD45, IL-10, IgD and CD5.

Advantageously, the invention relates to the method as defined above, for identifying a subpopulation of Epstein-Barr Virus (EBV) infected memory B cells, wherein the cells of the large population are labeled with at least the following markers: CD38, CD27, CD19, IgD and monoclonal or polyclonal antibodies directed to EBV.

More advantageously, the invention relates to the method as defined above, for identifying a subpopulation of regulating T cells, wherein the cells of the large population are labeled with at least the following markers: CD4, CD25, Foxp3 and antibodies directed to cytokines.

More advantageously, the invention relates to the method as defined above, for identifying a subpopulation of Human Immunodeficieny virus (HIV) infected CD4+ T cells, wherein the cells of the large population are labeled with at least the following markers: CD4, CD3, CD25 and antibodies directed to HIV antigens.

Advantageously, the invention relates to the method according to the above definition, wherein $\varepsilon = 10^{-1}$ and N= 10. Some advantageous results are obtained with N=500.

The invention also relates to a method for the in vitro diagnosis or the in vitro prognosis of pathologies, including cancers, vascular and immune pathologies, and infectious diseases, said method comprising the step of identifying a subpopulation of specific cells among a large population of cells, in an n-dimensional space, as defined previously.

Advantageously, the invention relates to a method for the in vitro diagnosis or the in vitro prognosis of pathologies, including cancers, vascular and immune pathologies, and infectious diseases, said method comprising the step of identifying a subpopulation of specific cells among a large population of cells, in an n-dimensional space, as defined in anyone of claims 1 to 11. In all the methods as defined above, a supplementary step can advantageously be carried out. This step e', occurring before step f consists to eliminate the cells that are considered as cells belonging to dense clusters, i.e. cells that belong to the larger clusters, advantageously larger clusters enlarged by p sliding regions.

The invention also relates to a computer program on an appropriated support for identifying a subpopulation of specific cells among a large population of cells, wherein said large population of cells is subject to steps a to b as set forth in anyone of claims 1-12, the computer program comprising means for causing a computer to carry out steps c. to g. as set forth in anyone of claims 1-12. When part or all of the functions of the present invention are realized using software, this software (computer program) can be provided in a form stored on a recording medium that can be read using a computer. For the present invention, a "computer-readable recording medium" is not limited to a recording medium in a portable format such as a floppy disk or CD-ROM, but can also be contained in an internal memory device within a computer such as various types of RAM and ROM, or in an external recording device fixed to a computer such as a hard disk.

The invention also relates to a kit comprising;

  a. n- reagents, said n- reagents to detect of the presence, the absence or the amount of n- different components of each cells of said large population, n being upper than or equal to 2,
  b. means for the detection of said n- different markers of cells, as defined above, and
  c. a computer program as defined above.

**Legends to the figures**

**Figures 1A-B** illustrate the detection of rare events with RARE on artificially generated data.
Figure 1A represents the dataset before treatment.
Figure 1B represents the dataset after treatment according to the invention: two rare events are present: one sparse and global and one dense and local.
**Figures 2A-C** illustrate the process according to the invention.

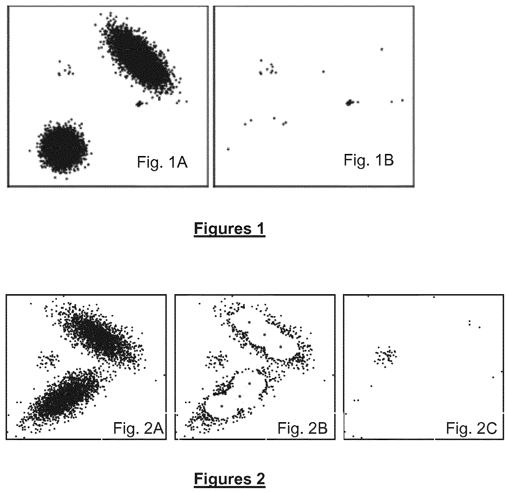

Figure 2A represents the original data: the rare event contains 1% of the entire data collection ($X$).
Figure 2B represents the data subset after eliminating the core of the dense regions ($X_{KEEP}$).
Figure 2C represents the rare events, after the elimination of the dense population ($X_{RARE}$).

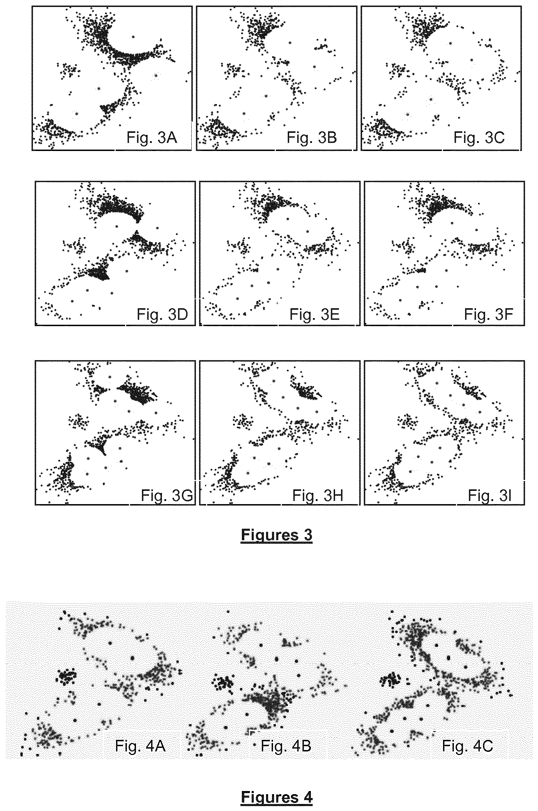

**Figure 3A-I:** Varying DMAX and KI in DenseKMeans considering the original data from Figure 2: (A,B,C) DMAX = 1:4, KI = 4; (D,E,F) DMAX = 1:2, KI = 6; (G,H,I) DMAX = 1, KI = 8.
Large points represent cluster centers. The initial, intermediary and final step for each case illustrate the convergence of cluster centers towards the core of the dense regions, eliminating the initial sensitivity of k-means to outliers.
**Figures 4:** Points in grey are eliminated through DenseSlide. The same combinations of DMAX and KI as in Figure 3 are used. In figure 4C, only 7 out of 8 clusters are left, one was eliminated because it did not fulfill the density condition (NI) in DenseKMeans.

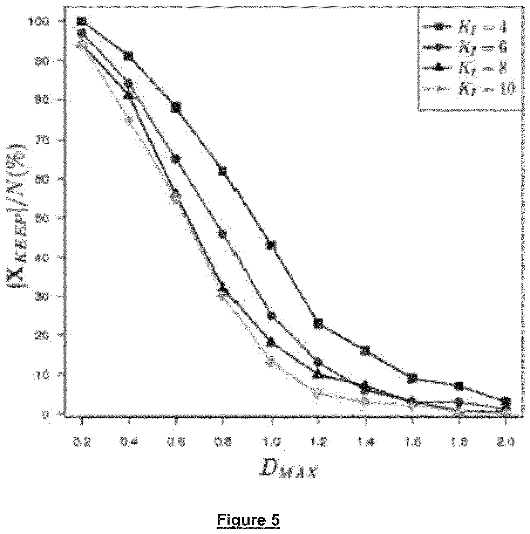

**Figure 5** represents Initialization of DMAX and KI for the artificial dataset from Figure 2.

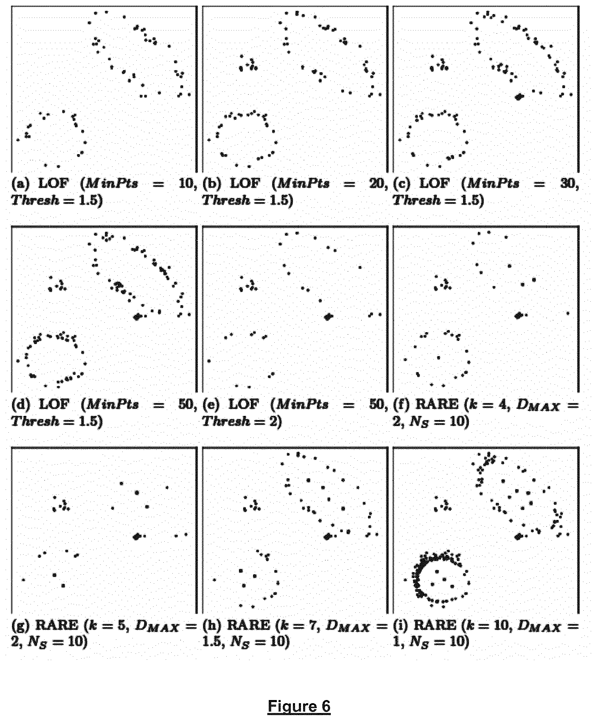

**Figure 6** represents examples of outlier detection by applying LOF (upper and middle panels) and RARE (lower panels) on artificially generated data. The large points in RARE indicate the cluster centers from DenseKMeans.

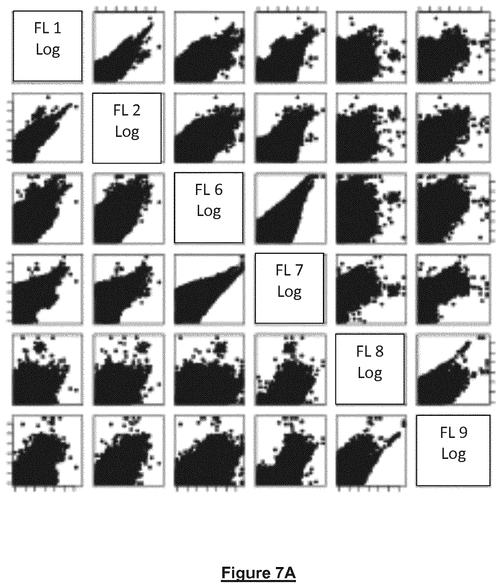

**Figure 7 A-B** represent the application of the method.
Figure 7A represents the original data according to the pair of detecting channels (FL1, FL2, FL6, FL7, FL8 and FL9)

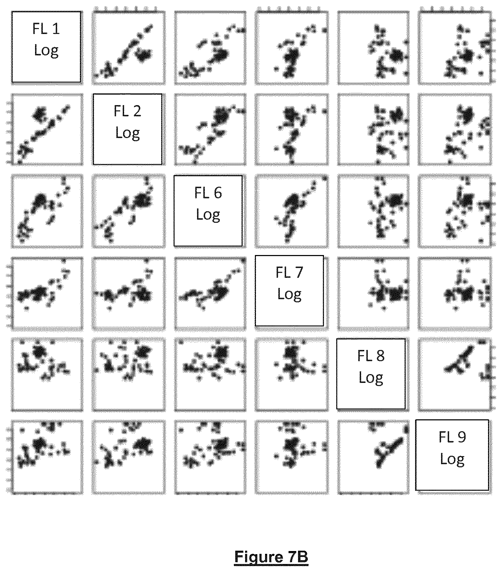

Figure 7B represents the rare events according to the pair of detecting channels (FL1, FL2, FL6, FL7, FL8 and FL9).

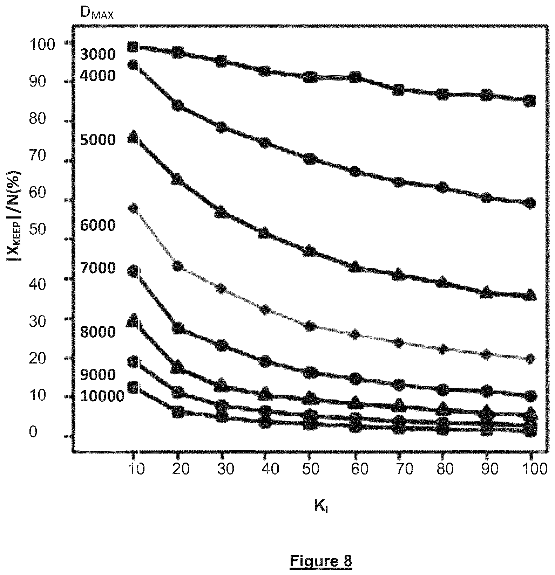

**Figure 8** represents the initialization of DMAX and KI for the flow cytometry dataset from Figure 7.

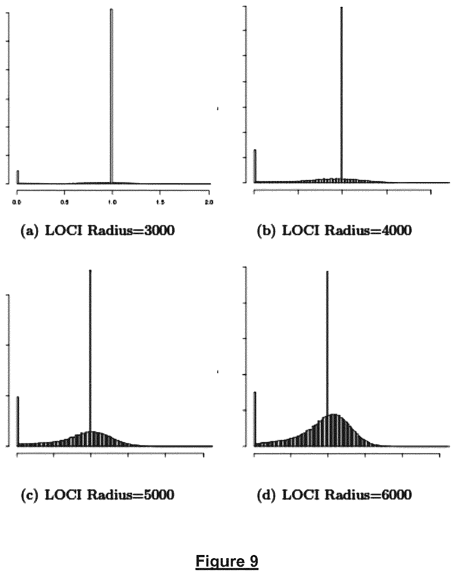

**Figure 9** represents the LOCI outlierness score for various radius values. All points in the rare event have a 1 score and cannot be identified as anomalies.

**EXAMPLES**

**Example 1:**

Introduction

An outlier is an observation which deviates so much from the other observations as to arouse suspicions that it was generated by a different mechanism" (Haw-kins1980). Similarly, a rare event - cluster of outliers (Rocke1996), clustered anomaly (Liu2010; Liu2012), anomaly collection (Dai2012), micro-cluster (Bae2012) - is a group of observations which deviates so much from the other groups of observations as to arouse suspicions that it was generated by a different mechanism.

The detection of rare events with a high recall, i.e. no false negatives, is intrinsic to those domains where the cost of missing rare events is significantly high. The most representative example is the medical domain where, for example, the cost of missing a pathological group of cells in a blood sample is significantly higher that the cost of classifying a healthy group of cells as pathological, i.e. favouring false positives over false negatives. Disease outbreaks in biosurveillance (Shmueli2010), bursts of clustered attacks (Liu2010) or groups of spammers/fraudulent reviewers in social media (Dai2012) are other examples of scenarios where the detection of rare events is prevailing over the cost of detecting them.

An anomaly - single or clustered - is an event considered as not normal with respect to a normal behaviour (Chandola2009). With any type of anomaly, the open issue is to define normality. For *single outliers,* normality is defined in terms of distance, distribution or neighborhood similarity with other data instances. For *spatial anomalies*, it is their occurrence in a specific region of the space that makes them abnormal. For *collective anomalies*, individual instances are normal but it is their co-occurrence that makes them anomalies. For *rare events*, it is their small relative size with respect to other data subpopulations that makes them anomalies. Contrary to collective anomalies, every instance contained in a rare event is an anomaly. Except its significantly small size, other data characteristics of a rare event, e.g. feature distribution or spatial positioning, carry no discriminant information with respect to normal data subpopulations. The inventors consider an example of rare events detection in Figure 1. The data distribution contains two normal populations of 10,000 points and two rare events: a sparser one of 10 points far from the normal populations, i.e. a *global* anomaly, and a denser one of 20 points close to one of the normal populations, i.e. a *local* anomaly. Figure 1(b) shows the output of an approach, RARE, isolating the rare events from the rest of the data.

Sharing common characteristics with both outliers and clusters, the detection of rare events lies at the frontier between outlier detection and strongly imbalanced/unbalanced clustering. Both clustering and outlier detection algorithms are generally prone to misclassify positive examples, i.e. rare events, as negative. Algorithms for unbalanced data have been mainly proposed in supervised scenarios (Tang2009) for classification problems in the presence of unbalanced training data where the problem is generally handled using resampling, cost-sensitive or one-class learning methods (Chawla2004). However, in unsupervised scenarios the problem is more difficult to handle as clustering algorithms generally tend to balance cluster sizes. K-means, for example, tends to reduce the variation in cluster sizes as a trade-off for a better accuracy (Xiong2006). In spectral clustering, both RatioCut and Ncut (Luxburg2007) put more emphasis on balancing clusters than on minimizing cut values. Both algorithms propose through the balancing constraints introduced to handle the outlier sensitivity of the initial MinCut solution. On the other hand, outli-er/anomaly detection algorithms are very effective at discovering single anomalies.

Different approaches (density-based, distance-based, distribution-based) have been proposed in the literature. The most common outlier detection algorithm, LOF (Breunig2000), Local Outlier Factor, outputs a list of top-k outliers according to an outlierness score that is obtained by comparing the local density of each point against the local density of the points in its neighbourhood. In LOF the quality of the result depends mainly on the construction of the neighbourhood (parameter MinPts).

In this paper the inventors address this gap between outlier detection and clustering methods. Given the main challenge to avoid false negatives, i.e. avoid missing true positives, the inventors propose a density-based backward or bottom-up approach, i.e. going from the most dense regions to the least dense ones. Common outlier detection methods use a forward or top-down approach, i.e. they take the top-k outliers according to an outlierness threshold score. The paper is organized as follows. Section 2 is dedicated to a literature review for finding rare events in large datasets. Section 3 introduces the RARE framework. The inventors first perform a clustering using DenseKMeans, a modified variant of k-means, designed to find and cluster only points that lie in dense regions of the space. In the second step, the inventors gradually augment the dense regions found by DenseKMeans using a density-based sliding region. As soon as the density inside the sliding region fails to fulfill a density condition, the inventors consider to have reached the border of the dense regions. Rare events lie outside these borders. In section 4 experiments on both synthetic and real data show that RARE is capable of finding rare events where other methods fail. The inventors close this paper with a conclusion and discussion on further perspectives in section 5.

2 Related work

Different approaches (Chandola2009; Ertoz2003; Ester1996; He2003; Liu2010; Liu2012; Papadimitriou2003; Zhu2010) in the literature have been proposed for the detection of rare events in large datasets. A few techniques approach it as cluster-based anomaly detection (Chandola2009): normal instances belong to large and dense clusters, while anomalies either belong to small or sparse clusters. Such methods rely on the output of a clustering algorithm. CBLOF (He2003) first performs a clustering, using any clustering method, and subsequently separates small from large clusters based on a predefined threshold. Using this threshold, it defines a Cluster-Based Local Outlier Factor (CBLOF) outlierness score by taking into account both the size of the cluster and the distance to the closest cluster center. Overall, the performance of such techniques relies strongly on the choice and quality of the initial clustering.

Employing explicit cluster size constraints is another solution (Zhu2010) that can be used to handle the detection of rare events in datasets. While the tendency in the literature is to concentrate on balancing clusters, this approach allows to generate a partitioning with different cluster sizes. It can be very helpful when an a priori knowledge on the size of each cluster in the data is known in advance. Still, only a few applications benefit from such a faithful information.

A third approach is to use or adapt single outlier detection algorithms and make them suitable for detecting micro-clusters of outliers. In LOF (Breunig2000) the detection of outlying clusters depends on the choice of the number of nearest neighbours MinPts that define the local neighbourhood. The detection of very small clusters requires a MinPts large enough to contain all the points in a cluster, i.e. larger than the size of the cluster. LOCI (Papadimitriou2003) defines a multi-granularity deviation factor (MDEF) and identifies outliers as those points whose neighbourhood size is significantly different than the neighbourhood size of their neighbours. Similarly to LOF, LOCI relies on an appropriate choice of the neighbourhood size, except that, contrary to LOF, it requires the maximum radius of the neighbourhood as input parameter.

Another different direction is to consider that normal instances belong to a cluster in the data, while outliers do not belong to any cluster (Chandola2009). This approach requires the use of methods (DBSCAN (Ester1996), SNN-based clustering (Ertoz2003)) that do not force every point to belong to one of the clusters. DBSCAN (Ester1996) is the most common density-based clustering algorithm. It builds clusters based on a novel notion of density reachability that allows clusters to be of different sizes and shapes. However the performance of DBSCAN is low when clusters are of different densities and both its run time complexity and memory are high $O(n^2)$.

A relatively recent concept - isolation - was proposed (Liu2008; Liu2010; Liu2012) as an alternative to the concepts of distance and density used in most outlier detection methods. The notion of isolation relies on the property of anomalies of being 'few and different'. The two methods, iForest (Liu2008; Liu2012) and SCiForest (Liu2010), that rely on this concept build in the training phase forests of t binary trees using sub-samplings of the data and compute in an evaluation step an anomaly score based on the path length of each point, defined as the path from the root of the tree to the node. While both methods are effective at discovering global clustered anomalies, i.e. clusters far apart from normal populations, only SCiForest is able to detect local clustered anomalies (Liu2012), i.e. clusters close to normal populations (the inventors presented both types of clustered anomalies in the example in Figure 1). However the high complexity of SCiForest in both training and evaluation stages, respectively $O(tT\psi(q\psi+log\psi+\psi))$ and $O(qnt\psi)$, where $\psi$ is the sampling size for building the iTrees and t the number of trees to build in the training phase, makes it adapted only in the presence of local clustered anomalies.

The RARE framework that the inventors propose in this paper proposes: 1) a backward approach to the detection of rare events by first identifying the normal/dense regions; 2) an approach designed to avoid false negatives and therefore accepting false positives, i.e. favour recall over precision; 3) a low complexity due to the use of a variant of k-means (linear, scalable); 4) a lower bound density-driven approach in the two steps of the framework that allow the detection of rare events.

### *3 The RARE framework*

The inventors describe in this section a two-stage framework for the detection of rare events in large datasets. Given a dataset **X** with N data points, the inventors define a rare event as in the following.

A rare event is a cluster of points of size $N_R$, where $N_R$ is significantly smaller than the total size of the dataset ($N_R$<<N).

When expressed in terms of the ratio $\varepsilon = \dfrac{N_R}{N}$ between the number of points in the rare event and the total number of points in the dataset, the above rare event condition becomes $\varepsilon$<<1. Very small values of $\varepsilon$, i.e. $\varepsilon$<$10^{-2}$, place the problem of abnormal events detection at the frontier between *outlier detection* and *strongly imbalanced clustering.*

### **The backward approach: an illustrative example**

The inventors illustrate the backward approach of RARE by means of an example in Figure 1. The inventors consider a dataset X with two main subpopulations and a rare event representing 1% of the whole dataset.

First, the inventors want to identify the core of the dense regions while handling two major issues at this stage: the scalability and the density. The inventors have no a priori knowledge on the number of subpopulations in the data. To handle the scalability issue the inventors choose to cluster the dataset using k-means [10] due to both its linear complexity and parallelization power. The density problem is then handled by modifying k-means so that only points that lie in dense regions are clustered. The inventors do this by changing in the re-assignment phase of k-means the way cluster centers are estimated, i.e. only points that lie at a maximum radius around cluster centers are used to recompute the centers. The radius-limited approach does not force all points to belong to one of the clusters, i.e. some points will be left unclustered. As the number of subpopulations is not known in advance, the inventors use a large initial number of clusters KI and let each population be modelled using multiple clusters. Figure 1(b) illustrates this first step of the analysis. The inventors use KI = 6 cluster centers in this example and plot the output of DenseKMeans, i.e. the points left unclustered after the first step, XKEEP .

In the second stage, the clusters that belong to the same population, i.e. they are adjacent as will be defined in Section 3.3, are merged to form connected components. In the example each group of 3 clusters forms a connected component. The two components are then gradually augmented using a Gaussian model to reach the border of the dense regions. Everything that is outside these borders, XRARE, is considered a rare event. The framework retrieves both true positives, i.e. the rare event, and false positives, i.e. points that lie close to the border of the dense regions or outliers (Figure 2(c)). False positives are the compromise that the inventors make for avoiding false negatives.

### 3.2 Dense regions clustering

The principle behind k-means relies on the minimization of a distance-based objective function that clusters the dataset X around K cluster centers. But this distance-based approach leaves k-means sensitive to density-related issues. The variant of k-means - DenseKMeans - that the inventors propose in the following addresses the density problem by bringing two modifications to the original algorithm:

$$\min \sum_{k=1}^{K} \sum_{x_i \in C_k} \|x_i - \mu_k\|^2$$

$$s.t. \, |C_k| > N_I$$

$$dist(x_i, CC_k) < D_{max}, \forall x_i \in C_k$$

1. initialization: choose cluster centers iteratively so that each new center is positioned at a minimum of DMAX distance from all the other centers and that each cluster center is assigned at least NI data points.
2. re-assignment: reestimate cluster centers using only points that are at a maximum of DMAX distance from one of the cluster centers and remove cluster centers that fall below the initial NI threshold during the re-assignment phase.

DenseKMeans is summarized in Table 1. The reestimation of cluster centers using only points that are at a maximum of $D_{MAX}$ distance from one of the cluster centers eliminates k-means' sensitivity to outliers - in case to rare events - as long as the radius $D_{MAX}$ is smaller than the distance to outliers. Moreover clusters $C_k$ that are not dense enough, card{Ck}< $N_I$, are discarded in the re-assignment phase.

These two modifications allow to restrict the region of the space considered by k-means to only dense regions and iteratively move cluster centers towards the core of the dense regions. Figure 3 illustrates a few examples with different parameter combinations DMAX vs. K: 1) DMAX = 1:4, KI = 4 (Figure 3 (a, b, c)); 2) DMAX = 1:2, KI = 6 (Figure 3 (d, e, f);) 3) DMAX = 1, KI = 8 (Figure 3 (g, h, i)). The output of this first stage of the algorithm divides the original dataset into two disjoint subsets X = XRMV U XKEEP : 1) XRMV = points falling within a maximum of DMAX distance from the final cluster centers, 2) XKEEP = points falling outside the region defined by the maximum DMAX distance from the final cluster centers. Using this approach, only points that are in dense regions are clustered.

3.3 Dense regions augmentation

DenseKMeans identifies the core of the dense regions using an initial number of clusters KI significantly higher than the actual number of clusters/data subpopulations. The radius-limited approach of DenseKMeans allows to define the cluster adjacency property as in the following:

**Definition 2** Two clusters defined by centers CCk and CCl are adjacent if the Euclidean distance between their centers is less than 2 * $D_{MAX}$:

$$\|CC_k, CC_l\|_2 < 2 * D_{MAX}$$

Among the final KF dense clusters found by DenseKMeans, adjacent clusters are merged to build connected components and provide a more faithful representation of the real data subpopulations.

A spherical model like the one used by k-means and DenseKMeans considers that the intrinsic dimensionality of the data is equal to the original dimensionality. However in real scenarios the intrinsic dimensionality of the data - especially locally, i.e. one data subpopulation/cluster - is rarely equal to the original dimensionality (Levina and Bickel 2005). To address this challenge, we treat the output of the spherical model by means of a model that is better adapted to handle the intrinsic dimensionality of the data. The most common is the Gaussian model. In the first step of the analysis, the spherical approach was preferred due to the scalability advantage of k-means. The use of the Gaussian mixture model in the first step would have required the estimation of K(D2 + D + 1) parameters for every value of K - as K is not known in advance. Even if parsimonious models, e.g. diagonal, can replace the full Gaussian model, the challenge to detect rare events is too sensitive and requires the use of a full model.

The subset XRMV allows to quickly estimate both the means $\mu j$ and covariance matrices $\Sigma j$ of the core dense regions defined by the connected components. These dense regions are augmented using a sliding region SR defined based on the Mahalanobis distance DM and an increase parameter $\varepsilon_S$. The sliding regions approach the border of the dense regions gradually and the process is repeated as long as a density condition is fulfilled, nbPoints(SR) > NS, i.e. the number of points inside the sliding region is larger than a predefined threshold NS. When the density inside the sliding region drops below this threshold, we consider to have reached the border of the dense regions. The algorithm for dense regions augmentation, DenseSlide, is summarized in Table 2 and a few examples for various combinations of parameters DMAX and KI are shown in Figure 4. The parameters for DenseSlide were $\varepsilon_S$ = 0.1 and NS = 10. The output of the algorithm returns the subset XRARE of positive examples.

4 Experiments

The behaviour and performance of RARE are illustrated in this section through experiments on both synthetic and real data. First an analysis and discus- sion on the choice of parameters are presented in Subsection 4.2. We use two artificially-generated datasets to show the behaviour of RARE for various parameter values. In Subsection 4.3 we test RARE on a large-scale real case application from the medical domain and compare it with two other outlier detection methods, LOF and LOCI.

4.1 Evaluation

We use Precision and Recall to evaluate the performance of the algorithms. Given our main challenge to avoid missing true positives, it is Recall that becomes the most important evaluation measure in this scenario. A high recall generally requires a low precision.

$$P = \frac{TP}{TP + FP} = \frac{TP}{|X_{RARE}|}$$

$$R = \frac{TP}{TP + FP} = \frac{TP}{N_{RS}}$$

where $|X_{RARE}|$ = the number of data points retrieved by the algorithm and NRS = the number of positives in the data, i.e. the size of the rare event.

4.2 Parameters in RARE

Throughout their evaluation, the inventors experimented with different values of the parameters and observed that, for a given application, the choice of the parameter values was consistent across different datasets. Values for DMAX and KI - parameters closely related - covering approximately 80 - 90% of the dataset in DenseKMeans ($X_{RMV}$) lead to good final results. This is due to the fact that the rare events represent significantly less than the rest of 10 - 20% of the whole dataset, allowing in the meantime for the core dense regions to be detected by DenseKMeans.

The minimal density NI required of a cluster in DenseKMeans depends on the size of the dataset N and the initial number of clusters KI and is fixed throughout this experiment to $NI = \frac{N}{10*K_I}$. The increasing parameter of the sliding region in DenseSlide was fixed to $\varepsilon_S = 10^{-2}$ and the minimal number of points required in the sliding region NS = 10. This way, out of the five parameters in the two steps of the framework the inventors are left with two free parameters: DMAX and $K_I$.

The inventors use the same synthetic dataset as in the illustrative example in Subsection 3.1 to discuss the choice of values for DMAX and KI. Figure 5 shows how the size of XKEEP evolves with these two parameters. As mentioned previously the inventors are interested in those value combinations between the two parameters that lead to a ratio $\frac{|K_{KEEP}|}{N}$ approximately in the range 10 - 20%. In figure 3, we showed a few examples with parameter combinations chosen in the above range. Generally, for approximately the same ratio $\frac{|K_{KEEP}|}{N}$, higher values for $K_I$ and lower values for $D_{MAX}$ are preferred as this reduces the risk of including the rare event into one of the clusters. The intermediary step in Figure 3 illustrates well how, due to the radius-limited approach of DenseKMeans, cluster centers converge towards the core of dense regions by eliminating *k*-means initial sensitivity to outliers and small groups of outliers/rare events.

Comparison with LOF.

The inventors reconsider the example from Figure 1. We show the output of both LOF (b-f) and our approach (g-j) using multiple parameter values. While LOF is prone at missing the rare events (a,b), RARE is prone at retrieving more points (j). This behaviour is in line with our challenge of avoiding the rare events, therefore favouring false positives over false negatives.

Parameters. RARE and LOF have similar parameters (Table 3). While LOF requires MinPts in the construction phase, RARE needs the two parameters $D_{MAX}$ and $K_I$ to find the dense regions. As already mentioned we choose generally combinations of these two parameters that cover approximately 80-90% of the data in DenseKMeans. Having two parameters adds flexibility but also more complexity. In DenseSlide RARE has two parameters $\varepsilon_S$ and $N_S$, the growing rate of the sliding region and the minimal density required ($\varepsilon_S$ is generally fixed to either $10^{-1}$ or $10^{-2}$). Their influence is equivalent to the cutting threshold in LOF, but it is the approach that is different: LOF has a top-down approach while RARE has a bottom-up approach. The bottom-up approach is however more suitable in scenarios where avoiding false negatives is the priority.

4.3 A real case: flow cytometry

In flow cytometry each cell is characterized by fluorescence levels in response to cell markers, i.e. attributes. Nowadays flow cytometers can count up to tens of millions of cells representing normal cell populations found in any healthy patient, such as lymphocytes or monocytes. In patients presenting a blood pathology, the blood samples also contain micro-clusters of cells with abnormal signatures, i.e. abnormal combinations of cell marker fluorescence levels. The human detection of these rare events is performed visually by sequentially inspecting two-dimensional spaces, i.e. combinations of two markers. This approach leads to a very high inter-variability (17-44%) (Bashashati 2009) among research laboratories between what defines an abnormal cell population and is sensitive to complex multivariate relationships.

Figure 7 shows a flow cytometry sample of 752,987 cells containing a rare event of 30 cells. The rare event initially visible only on the FL.8.Log attribute, emerges also on the other attributes after processing with RARE. For this experiment we fixed $NI = \frac{N}{100 * K_I}$ (we know the rare event is signficantly smaller that the total size of the dataset). Figure 8 shows the percentages of data covered by DenseKMeans in the first step of the algorithm for various values of the parameters $D_{MAX}$ and $K_I$. For the rest of the experiments in this paper, we choose the free parameters $D_{MAX}$ and $K_I$ that guarantee the ratio $\frac{|K_{KEEP}|}{N}$ across the different blood samples, i.e. $D_{MAX}$ and $K_I=40$.

Experiment 1: Varying NR. The inventors first wish to test the performance of RARE for varying levels of unbalancedness. In this purpose the inventors will keep the total size of the dataset fixed and vary the size of the rare event - which is an indicator of the phase of the pathology. On the biological side, this experiment was performed by injecting grown cells from a blood pathology into a cell sampling of a healthy patient. The size of the rare population injected was of {f5; 10; 20; 50; 100; 500}. Due to machine error, a difference appears between the number of injected cells and the actual size of the rare cell population found in the blood samples, i.e. positive examples (corresponding to a pathology signature in flow cytometry): NRS = {4; 14; 17; 31; 82; 359}. The whole dataset contained N = NH + NRS cells, where NH $\approx$ 700:000 cells. The parameters for DenseSlide were chosen: $\varepsilon_S = 10^{-1}$ and NS = 10.

The results in Table 4 show an excellent performance for RARE which finds almost all positive examples, i.e. true positives TP (column 3), among the positive examples NRS found with the signature provided by domain experts (column 5). The size of the false positives FP returned by RARE (column 4) depends mainly on the size and structure of the original dataset, i.e. FP remains relatively constant with increasing TP. We also observe that the recall is relatively high and the precision increases with the size of the rare event. Comparison with LOF and LOCI. In Table 5 we analysed the blood samples using LOF (Breunig et al 2000) and show the results for different LOF score threshold values. The analysis was performed on a sampling of 100K cells containing the rare event and we chose the parameter MinPts equal to NR and thus higher than the number of cells in the rare event, i.e. this is necessary for the detection of micro-clusters in LOF. We chose three threshold values: 2, 1.5 and a third value corresponding to the minimum LOF score retrieving all cells in the rare event. We observe that for the same recall for LOF and RARE, LOF has a significantly lower precision (e.g. it needs to retrieve approximately 80% of the dataset for NR = 20 and 60% for NR = 100 for a high recall). For a threshold of LOF>1.5, the precision is always lower than RARE for a much lower recall. We applied LOCI (Papadimitriou et al 2003) on samples with each of the NR = f5; 10; 20; 50; 100; 500g. We used various values of the maximum radius in LOCI {3000; 4000; 5000; 6000g} but obtained every time a 1 score for the points in the rare event (a few examples of LOCI score frequency are presented in Figure 9). A 1 score in both LOF and LOCI indicate inliers and the rare event could not be detected. For values of the radius > 6000 we ran into memory problems.

Experiment 2: Cancer and intracranial aneurysm. The inventors tested their framework on real patient blood samples (4 for cancer and 6 for intracranial aneurysm). The samples were significantly larger than the biological benchmark (2-5 million cells). The parameters of DenseKMeans were the same as for the biological benchmark: DMAX = 8000, KI = 40 to guarantee the ratio $\frac{|K_{KEEP}|}{N} \approx 10 - 20\%$ across the different blood data samples. In DenseSlide the inventors chose a smaller increasing parameter for the sliding region $\varepsilon_S = 10^{-2}$ to account for the sensitivity of real data, i.e. slower approach of the border of the dense regions. A high recall $\frac{TP}{N_{RS}}$ demands a low precision $\frac{TP}{|X_{RARE}|}$ Still the ratio $\frac{|X_{RARE}|}{N}$ is very small (order of $10^{-2}$-$10^{-3}$) which guarantees a very good isolation of rare events with a high recall. Due to the low recall we increases the stopping (cutting) parameter NS = {50; 100; 500}. With an increase in NS DenseSlide stops earlier, with leads to an increase in recall and a decrease in precision. The results show that the rare events were more easily isolated for intracranial aneurysm blood samples than for the cancer samples.

Experiment 3 : Comparison with DBSCAN and LOF

A comparison of the parameters required by the three methods is presented in Table 8 .While LOF requires only one parameter - MinPts - in the construction phase, DBSCAN and RARE both require two parameters, thus adding more flexibility but also more complexity to the model. Both RARE and LOF require a stop- ping criteria while DBSCAN considers all points left unclustered as noise. Rare events will often fall in the noise category with DBSCAN (as shown in the next experiment). RARE uses two parameters - $\varepsilon_S$ and NS , the growing rate of the sliding region and the minimal density ($\varepsilon_S$ is generally fixed to either $10^{-1}$ or $10^{-2}$) - to define the stopping criteria. Their influence is equivalent to the cutting threshold in LOF, but it is the approach that is different: LOF has a top-down approach while RARE has a bottom-up approach. The bottom-up approach is preferred in scenarios where avoiding false negatives is the priority.

In Table 9, the inventors analyzed a data sample chosen at random from the second experiment with a medium rare event (752987 samples and 31 positive examples) using various parameter values for the three methods. The inventors compute the number of true positives (TP) and false positives (FP) retrieved by the algorithms. Both RARE and DBSCAN have a high recall (generally 100%) while RARE has a significantly higher precision than DBSCAN. In DBSCAN for most parameter values the rare event is left unclustered and belongs to the subset classified as noise 5 - except in the two cases where a fraction of the rare event clusters separately in a small cluster (14 and 25 points). While DBSCAN requires the MinPts parameter to be lower than the size of the rare event for a relatively good performance, LOF on the contrary requires the MinPts parameter higher than the size of the rare event, i.e. this is necessary for the detection of micro- clusters in LOF. While DBSCAN requires no stopping criteria, in LOF the inventors need to choose either the cutting threshold value or the number of outliers. The inventors use here two cutting threshold values for each value of MinPts in LOF and indicate the number of false positives in each case. The two values were chosen so that the vast majority of the rare event has an LOF outlierness score in the range bounded by the two values.

5 Conclusion

The inventors proposed here a backward approach framework to isolate rare events in large datasets. The size of these events makes their detection difficult by both clustering and outlier detection algorithms as both tend to misclassify true positives as false negatives. The RARE framework targets applications where recall prevails over precision, e.g. medicine, emergent roles in social networks. The new variant of k-means was proposed to handle the scalability and density issues in this type of problems and the sliding region is designed to avoid false negatives. The inventors showed that the main parameters DMAX and KI can be chosen to guarantee a 10 - 20% cover percentage in the first step with preference given to smaller DMAX and larger KI. The complexity is largely dominated by the complexity of DenseKMeans which is linear and could be improved by parallelization.

**Bibliography of the example**

[1] D.H. Bae, S. Jeong, S.W. Kim and M. Lee. Outlier detection using centrality and center-proximity. In Proceedings of CIKM, 2012.
[2] A. Bashashati and R. Brinkman. A survey of ow cytometry data analysis methods. In Advances in Bioinformatics, 2009.
[3] M. Breunig, H.P. Kriegel, R.T. Ng and J. Sander. LOF: identifying densitybased local outliers. In Proceedings of ACM SIGMOD, 2000.
[4] V. Chandola, A. Banerjee and V. Kumar. Anomaly detection: a survey. ACM Computing Surveys, 41. 2009.

[5] L. Ertöz, M. Steinbach and V. Kumar. Finding clusters of di_erent sizes, shapes and densities in noisy, high-dimensional data. SDM, 2003.

[6] M. Ester, H.P. Kriegel, J. Sander and X. Xu. A density-based algorithm for discovering clusters in large spatial databases with noise. In Proceedings of ACM SIGKDD, 1996.

[7] Z. He, X. Xu and S. Deng. Discovering cluster-based local outliers. Pattern Recognition Letters 24, 2003.

[8] E. Levina and P.J. Bickel. Maximum likelihood estimation of intrinsic dimension. Advances in Neural Information Processing Systems, 17, 2005.

[9] U. von Luxburg. A tutorial on spectral clustering. Statistics and Computing, 17, 2007.

[10] J.B. MacQueen. Some methods for classification and analysis of multivariate observations. In Proceedings of the Fifth Berkeley Symposium on Mathematical Statistics and Probability, 1967.

[11] S. Papadimitriou, H. Kitagawa, P. Gribbons and C. Faloutsos. LOCI: Fast outlier detection using the local correlation integral. In Proceedings of ICDE,2003.

[12] Y. Tang, Y. Q. Zhang, N. W. Chawla and S. Krasser. SVMs for highly imbalanced classification. IEEE Transactions on Systems, Man and Cybernetics, 39:281-288, 2009.

[13] H. Xiong, J. Wu, and J. Chen. K-means clustering versus validation measures: a data distribution perspective. KDD, 2006.

[14] S. Zhu, D. Wang, and T. Li. Data clustering with size constraints. Knowledge-Based Systems, Elsevier, 23:883-889, 2010.

TABLES

Table 1: DenseKMeans.

**Input: X** = {$x_1$}, i = 1..$N$, $x_1 \in$ **R**$^D$
$\qquad$ $K_I$ - initial number of clusters
$\qquad$ $N_I$ - minimum number of points (density)
$\qquad$ $D_{MAX}$ - radius
**Output: CC** = {$CC_k$}, $k$ = 1..$K_F$ - final cluster centers
$\qquad$ **X**$_{KEEP}$ - the subset of points left unclustered
$\qquad$ **X**$_{RMV}$ - the subset of points clustered

**Initialization:**
**1':** Choose cluster centers CC iteratively so that they are further than $D_{MAX}$ one from each other:
$$\|CC_k, CC_1\|_2 > D_{MAX}, \forall k,l = 1.K_I$$
**2':** Check the density condition: $card\{C_k\} > N_I$
**3':** Repeat steps 1' and 2' until convergence: all $K_I$ centers are assigned at least $N_I$ points.

**DenseKMeans:**
**1":** Select all points **X**$_{KEEP}$ that are further than $D_{MAX}$ from all centers:
$$min(x_1, CC_k) > D_{MAX}$$
**2":** Reestimate cluster centers using **X**$_{RMV}$ = **X** \ **X**$_{KEEP}$
**3":** If a cluster center falls under the initial $N_I$ threshold ($card\{C_k\} < N_I$) remove it.
**4":** Repeat steps 1"-3" until convergence: a maximum number of iterations is reached or centers do not change significantly.

Table 2: DenseSlide.

**Input: X**$_{KEEP}$, **X**$_{RMV}$, **CC** - output of DenseKMeans
$\qquad$ $\varepsilon_S$ - increase parameter for the sliding region
$\qquad$ $N_S$ - number of points in the sliding region
**Output: X**$_{RARE}$ - rare events

**Connected components:**
**1':** Build the graph $G$ = (CC, $E$) using the cluster adjacency property.
**2':** Find connected components $G_j$ in C.

(continued)

3': Use $\mathbf{X}_{RMV}$ to model $G_j$ as $\mathcal{N}(\mu_j, \Sigma_j)$.

**Sliding Region:**

1": Initialize $\mathbf{X}_{RARE}=\mathbf{X}_{KEEP}$.

2": For each $G_j$ compute the Mahalanobis distance:

$$D_M^j = \sqrt{(\mathbf{X}_{RARE} - \mu_j)^T \Sigma_j^{-1} (\mathbf{X}_{RARE} - \mu_j)}$$

3": Eliminate points from $\mathbf{X}_{RARE}$ that are closer to one of the component centers than the farthest point from

$\mathbf{X}_{RMV}$: $D_M^j(x_i) > D_{max}^j$.

4": Create a moving sliding region $S_R(D_{max}^j, \epsilon_S)$ around each component $\mathcal{N}(\mu_j, \Sigma_j)$.

5": Eliminate points from $\mathbf{X}_{RARE}$ inside $S_R$.

6": Repeat steps 4" and 5" as long as the density condition is respected: $nbPoints(S_R) > N_S$.

**Table 3** Parameters in RARE vs. LOF.

| RARE | $D_{MAX}$&$K_I$ (DenseKMeans) | $\varepsilon_S$&$N_S$ (DenseSlide) | Bottom-up (backward) |
|---|---|---|---|
| LOF | *MinPts* (neighbourhood) | *Threshold* or top-*k* | Top-down (forward) |

**Table 4** RARE on five samples for each of the varying $N_R$ = {5, 10, 20, 50, 100, 500}

| $N_R$ | $N$ | $\frac{|\mathbf{X}_{KEEP}|}{N}$ (%) | $|\mathbf{X}_{RARE}|$ | *TP* | *FP* | P | R | $N_{RS}$ |
|---|---|---|---|---|---|---|---|---|
| 0 | 151,388 | 7.7% | 64 | **5** | 59 | 7.8% | 100% | **5** |
| 5 | 646,149 | 8.1% | 42 | **4** | 38 | 9.5% | 100% | **4** |
| 10 | 780,988 | 7.6% | 54 | **13** | 39 | 24% | 92.8% | **14** |
| 20 | 757,234 | 7.5% | 70 | **17** | 53 | 24.2% | 100% | **17** |
| 50 | 752,987 | 7.4% | 65 | **30** | 35 | 46.1% | 96.7% | **31** |
| 100 | 760,842 | 7.2% | 132 | **80** | 52 | 60.6% | 97.5% | **82** |
| 500 | 718,743 | 7.7% | 415 | **358** | 57 | 86.2% | 99.7% | **359** |
| 0 | 696,465 | 10.9% | 102 | **14** | 88 | 13.7% | 100% | **14** |
| 5 | 731,576 | 11.0% | 98 | **9** | 89 | 9.1% | 75% | **12** |
| 10 | 720,945 | 9.9% | 114 | **14** | 100 | 12.2% | 100% | **14** |
| 20 | 484,285 | 10.5% | 129 | **25** | 104 | 19.3% | 96.1% | **26** |
| 50 | 630,341 | 10.4% | 40 | **35** | 5 | 87.5% | 97.2% | **36** |
| 100 | 676,745 | 10.2% | 142 | **69** | 77 | 48.5% | 98.5% | **70** |
| 500 | 516,981 | 11.2% | 541 | **366** | 175 | 67.6% | 98.6% | **371** |
| 0 | 671,582 | 10.1% | 94 | **8** | 86 | 8.5% | 100% | **8** |
| 5 | 707,535 | 10.8% | 100 | **7** | 93 | 7% | 100% | **7** |
| 10 | 714,081 | 10.2% | 135 | **13** | 122 | 9.6% | 100% | **13** |
| 20 | 621,155 | 11.8% | 155 | **11** | 144 | 7% | 100% | **11** |
| 50 | 599,851 | 10.2% | 144 | **26** | 118 | 18% | 100% | **26** |
| 100 | 711,801 | 10.5% | 204 | **84** | 120 | 41.1% | 100% | **84** |
| 500 | 993,671 | 10.7% | 552 | **312** | 240 | 56.5% | 100% | **312** |
| 0 | 737,997 | 12.1% | 253 | **9** | 244 | 3.5% | 90% | **10** |
| 5 | 711,130 | 10.5% | 118 | **10** | 108 | 8.4% | 100% | **10** |
| 10 | 707,199 | 10.3% | 113 | **11** | 102 | 9.7% | 100% | **11** |
| 20 | 702,362 | 10.4% | 104 | **16** | 88 | 15.3% | 100% | **16** |

(continued)

| $N_R$ | $N$ | $\dfrac{|\mathbf{X}_{KEEP}|}{N}$(%) | $|\mathbf{X}_{RARE}|$ | TP | FP | P | R | $N_{RS}$ |
|---|---|---|---|---|---|---|---|---|
| 50 | 620,829 | 10.2% | 159 | **29** | 130 | 18.2% | 100% | **29** |
| 100 | 674,316 | 10.2% | 165 | **70** | 95 | 42.4% | 100% | **70** |
| 500 | 658,590 | 10.1% | 593 | **336** | 257 | 56.6% | 99.7% | **337** |
| 0 | 602,814 | 9.9% | 131 | **12** | 119 | 9.1% | 100% | **12** |
| 5 | 618,192 | 10.5% | 93 | **9** | 84 | 9.6% | 90% | **10** |
| 10 | 703,027 | 9.5% | 122 | **13** | 109 | 10.6% | 100% | **13** |
| 20 | 701,580 | 9.8% | 112 | **16** | 96 | 14.2% | 94.1% | **17** |
| 50 | 381,654 | 11.0% | 111 | **25** | 86 | 22.5% | 100% | **25** |
| 100 | 719,439 | 11.5% | 149 | **64** | 85 | 42.9% | 98.4% | **65** |
| 500 | 648,391 | 10.1% | 520 | **317** | 203 | 60.9% | 100% | **317** |

**Table 5** LOF for varying $N_R$.

| $N_R$ (MinPts) | LOFscores | $N_{retrieved}$ | TP | P | R | $N_{RS}$ |
|---|---|---|---|---|---|---|
| 5 | >2 | 39 | 0 | 0% | 0% | **4** |
| | >1.5 | 1170 | 1 | $8.5*10^{-2}$% | 25% | |
| | >1.24 | 7,801 | **4** | $5.1*10^{-2}$% | 100% | |
| 10 | >2 | 33 | 0 | 0% | 0% | **14** |
| | >1.5 | 665 | 4 | 0.6% | 28.5% | |
| | >1.09 | 25,670 | **14** | $5.4*10^{-2}$% | 100% | |
| 20 | >2 | 35 | 0 | 0% | 0% | **17** |
| | >1.5 | 571 | 1 | $1.7*10^{-1}$% | 5.8% | |
| | >1.00 | 80,292 | **17** | $2.1*10^{-2}$% | 100% | |
| 50 | >2 | 49 | 1 | 2% | 3.2% | **31** |
| | >1.5 | 697 | 3 | 0.4% | 9.6% | |
| | >1.27 | 5,985 | **31** | 0.5% | 100% | |
| 100 | >2 | 45 | 0 | 0% | 0% | **82** |
| | >1.5 | 821 | 2 | $2.4*10^{-1}$% | 2.4% | |
| | >1.03 | 58,268 | **82** | $1.3*10^{-1}$% | 100% | |
| 500 | >2 | 95 | 0 | 0% | 0% | **359** |
| | >1.5 | 2,180 | 0 | 0% | 0% | |
| | >1.09 | 38,840 | **359** | $9.2*10^{-1}$% | 100% | |

**Table 6** Cancer.

| Pat. | $N$ | $\dfrac{|\mathbf{X}_{KEEP}|}{N}$(%) | $N_S$ | $|\mathbf{X}_{RARE}|$ | TP | FP | P | R | $N_{RS}$ |
|---|---|---|---|---|---|---|---|---|---|
| C1 | 2,470,042 | 20.4% | 50 | 7,117 | **2** | 7,115 | $2.8*10^{-2}$% | 28.5% | **7** |
| | | | 100 | 14,113 | **4** | 14,109 | $2.8*10^{-2}$% | 57.1% | |
| | | | 500 | 91,337 | **7** | 91,330 | $7.6*10^{-3}$% | 100% | |
| C2 | 3,413,325 | 36.7% | 50 | 10,787 | **24** | 10,763 | $2.2*10^{-1}$% | 96% | **25** |
| | | | 100 | 14,311 | **24** | 14,287 | $1.6*10^{-1}$% | 96% | |
| | | | 500 | 28,130 | **25** | 28,105 | $8.8*10^{-2}$% | 100% | |

(continued)

| Pat. | $N$ | $\frac{|\mathbf{X}_{KEEP}|}{N}$(%) | $N_S$ | $|\mathbf{X}_{RARE}|$ | TP | FP | P | R | $N_{RS}$ |
|---|---|---|---|---|---|---|---|---|---|
| C3 | 5,989,247 | 16.2% | 50<br>100<br>500 | 6,654<br>36,984<br>95,403 | **1**<br>**31**<br>**41** | 6,653<br>36,953<br>95,362 | $1.5 * 10^{-2}$%<br>$8.3 * 10^{-2}$%<br>$4.3 * 10^{-2}$% | 2.4%<br>75.6%<br>100% | **41** |
| C4 | 5,959,464 | 15.6% | 50<br>100<br>500 | 3,071<br>4,241<br>19,479 | **24**<br>**25**<br>**32** | 3,047<br>4,216<br>19,447 | $7.8 * 10^{-1}$%<br>$5.9 * 10^{-1}$%<br>$1.6 * 10^{-1}$% | 54.5%<br>56.8%<br>72.7% | **44** |

**Table 7** Intracranial aneurysm.

| Pat. | $N$ | $\frac{|\mathbf{X}_{KEEP}|}{N}$(%) | $N_S$ | $|\mathbf{X}_{RARE}|$ | TP | FP | P | R | $N_{RS}$ |
|---|---|---|---|---|---|---|---|---|---|
| A1 | 2,524,916 | 22.1% | 50<br>100 | 6,727<br>8,987 | **15**<br>**15** | 6,712<br>8,972 | $2.2 * 10^{-1}$%<br>$1.6 * 10^{-1}$% | 100%<br>100% | **15** |
| A2 | 4,130,539 | 23.2% | 50<br>100 | 3,615<br>5137 | **6**<br>**6** | 3,609<br>5131 | $1.6 * 10^{-1}$%<br>$1.1 * 10^{-1}$% | 75%<br>75% | **8** |
| A3 | 4,595,598 | 18.5% | 50<br>100 | 3,986<br>6,252 | **12**<br>**16** | 3,974<br>6,236 | $3 * 10^{-1}$%<br>$2.5 * 10^{-1}$% | 70.5%<br>94.1% | **17** |
| A4 | 1.895,261 | 15.7% | 50<br>100 | 6,971<br>13,397 | **23**<br>**23** | 6,948<br>13,374 | $3.2 * 10^{-1}$%<br>$1.7 * 10^{-1}$% | 92%<br>92% | **25** |
| A5 | 1,899,278 | 15% | 50<br>100 | 4,698<br>7,030 | **21**<br>**21** | 4,677<br>7,009 | $4.4 * 10^{-1}$%<br>$2.9 * 10^{-1}$% | 100%<br>100% | **21** |
| A6 | 3,039,332 | 17.7% | 50<br>100 | 6,244<br>10,906 | **18**<br>**18** | 6,226<br>10,888 | $2.8 * 10^{-1}$%<br>$1.6 * 10^{-1}$% | 100%<br>100% | **18** |

**Table 8. Parameters in RARE, DBSCAN and LOF**

| Method | Model parameters | Stopping criteria | Approach |
|---|---|---|---|
| RARE | (DMAX,KI) | ($\varepsilon$S,NS) | Bottom-up (backward) |
| DBSCAN | ($\varepsilon$,MinPts) | - | Bottom-up |
| LOF | MinPts | Threshold or top-k | Top-down (forward) |

Table 9 Comparison between RARE ,DBSCAN and LOF. The parameter values in the second column correspond to the respective parameters of each method from the first column.

| Method | Parameters | TP | FP |
|---|---|---|---|
| RARE($D_{MAX}$, $K_I$, $\varepsilon_S$, $N_S$) | (6000, 80, 0.1, 10) | 31 | 193 |
| | (6000, 100, 0.1, 10) | 31 | 48 |
| | (7000, 40, 0.1, 10) | 31 | 43 |
| | (7000, 60, 0.1, 10) | 31 | 60 |
| | (7000, 80, 0.1, 10) | 31 | 57 |
| | (7000, 100, 0.1, 10) | 30 | 40 |
| | (8000, 20, 0.1, 10) | 31 | 184 |
| | (8000, 40, 0.1, 10) | 31 | 60 |
| | (8000, 60, 0.1, 10) | 31 | 22 |
| | (9000, 10, 0.1, 10) | 31 | 284 |
| | (9000, 30, 0.1, 10) | 31 | 48 |
| | (9000, 50, 0.1, 10) | 31 | 35 |
| | (10000, 10, 0.1, 10) | 31 | 51 |
| | (10000, 30, 0.1, 10) | 31 | 35 |
| DBSCAN($\varepsilon$, MinPts) | (5000, 10) | 31 | 1286 |
| | (5000, 20) | 31 | 1998 |
| | (5000, 30) | 31 | 2703 |
| | (6000, 10) | 31 | 457(14) |
| | (6000, 20) | 31 | 699 |
| | (6000, 30) | 31 | 934 |
| | (7000, 10) | 31 | 197(25) |
| | (7000, 20) | 31 | 331 |
| | (7000, 30) | 31 | 396 |
| LOF(MinPts, Threshold) | (30, 1) | 31 | 589039 |
| | (30, 1.1) | 3 | 132890 |
| | (50, 1.5) | 31 | 2133 |
| | (50, 1.6) | 8 | 945 |
| | (100, 2) | 31 | 230 |
| | (100, 2.5) | 3 | 54 |
| | (150, 2.1) | 31 | 206 |
| | (150, 2.7) | 3 | 43 |

## Example 2

### Experimental data

### Material & method

Patients and blood samples collection

Peripheral blood samples were collected using EDTA-containing tubes from healthy donor, patients with intracranial aneurysm and patients with colorectal cancer.

Flow cytometry

Cell analysis

For CEC analysis, blood samples were prepared with an hypotonic lysis wash procedure. 4 mL of blood were transferred into a 50 mL tube and a solution of ammonium chloride 0.15 M was added at 1V/5V for red blood lysis. After 5 min at 4°C, the suspension was centrifuged at 400 x g for 5 min at 4°C, the supernatant was removed and the pellet was washed with 20 mL of NH4Cl solution and the suspension was centrifuged immediately (400 × g, 4°C and 5 min).

The pellet was washed with a solution of RPMI 1640 and after centrifugation, cells were incubated in darkness at room temperature (RT) during 15 min with a mixture of the following monoclonal antibodies: 5 μL of pacific-blue (PB) conjugated CD31 (clone 5.6E; Beckman-Coulter, USA); 10 μL of krome-Orange (KO) conjugated CD45 (clone J.33; Beckman-Coulter); 5 μL of fluorescein isothiocyanate (FITC) conjugated CD34 (clone 581; Beckman-Coulter); 5 μL of phycoerythrin (PE) conjugated CD105 (clone 43A4E1; Miltenyi Biotec GmbH, Germany); 10 μL of 7-aminoactinomycin D (Beckman Coulter); 5 μL of phycoerythrin cyanine-7 (PC7) conjugated CD309 (clone KDR-1; Beckman Coulter), and 5 μL of allophycocyanin (APC) conjugated CD146 (clone 541-10B2, Miltenyi Biotec).

After incubation, cells were washed with a phosphate buffer solution supplemented with 2% FCS (400 × g, 5 min, 4°C). After removal the supernatant, the cells were resuspended in 1 mL of PBS.

Samples were acquired on a CyAn flow cytometer with Summit 6.1 software (Beckman Coulter).

HUVECs culture

Human Umbilical Vein Endothelial Cells (HUVECs) were used to analyze the sensitivity and the repeatability of RARE by an adding of HUVECs by a cell sorter. HUVEC-c (Promocell GmbH, Germany) are cultured in a 75 cm$^2$ flask in 27 mL of an Endothelial cell growth medium (Promocell) at 37°C, 5% $CO_2$ with a plating density at 5000 -10000 cells per cm$^2$.

Once they have reached 70-90% confluency, 7.5 mL of Hepes BSS solution (Promocell) was added of vessel surface to wash the cells. The Hepes BSS was aspirated, 7.5 mL of Trypsin/EDTA solution was added 2 min to detach HUVECs and 7.5 mL of FCS was added to neutralize Trypsin. The suspension was centrifuged at 220 × g for 5 min and the pellet was resuspended in 90 μL of PBS.

HUVECs Sorting

HUVECs were stained with the same mixture for CEC detection and acquired on a MofLo Astrios cell sorter (Beckman Coulter). After elimination of doublets, the sorting was based on 2 parameters: FCS and SSC. 0, 5, 10, 20, 50, 100 or 500 HUVECs were distributed in standard 5-mL flow cytometry tubes containing 10$^6$ peripheral blood mononuclear cells stained with the same monoclonal antibodies.

The suspension containing PBMCs and HUVECs were analyzed on CyAn flow cytometer.

CECs/HUVECs signature

With the multiplicity of dot plot, all cell markers of interest are analyzed and their thresholds are determined. With this gating strategy, the inventors can define a signature for the population of interest.

To apply this signature in the RARE approach, the signature must be converted in the informatics language.

X and Y values correspond to a channel when data are analyzed with a cytometry software but in the informatics language, the 1023 channel are distributed in 65532 values. So, to generate the signature for RARE, the inventors must apply this equation:

$$\text{RARE threshold} = (\text{software threshold} * 65532) / 1023.$$

A RARE signature for identify the population of interest is generated.

Results

To measure the sensibility and the reproducibility of CEC measurements by flow cytometry, 0, 5, 10, 20, 50, 100 and 500 HUVECs were sorted on Moflo Astrios and mixed with 10$^6$ peripheral blood mononuclear cells derived from a single blood draw stained with the same multicolor panel. These enumerations were performed in quintuplicate. Cells were analyzed on CyAn flow cytometer and data analysis was performed on Kaluza™ software (Beckman Coulter) and on RARE framework.

Results are detailed in the following table :

| | | Counted cells | | | | | | | | | |
|---|---|---|---|---|---|---|---|---|---|---|---|
| | | Kaluza™ | | | | | RARE framework | | | | |
| | | 1 | 2 | 3 | 4 | 5 | 1 | 2 | 3 | 4 | 5 |
| Sorted cells | 0 | 3 | 0 | 0 | 0 | 0 | 5 | 14 | 8 | 9 | 12 |
| | 5 | 3 | 3 | 2 | 4 | 5 | 4 | 9 | 7 | 10 | 9 |
| | 10 | 9 | 6 | 6 | 4 | 8 | 13 | 14 | 13 | 11 | 13 |
| | 20 | 16 | 12 | 10 | 12 | 10 | 17 | 25 | 11 | 16 | 16 |
| | 50 | 25 | 29 | 16 | 21 | 22 | 30 | 35 | 26 | 29 | 25 |
| | 100 | 68 | 50 | 67 | 56 | 56 | 80 | 69 | 84 | 70 | 64 |
| | 500 | 340 | 303 | 256 | 305 | 295 | 358 | 366 | 312 | 336 | 317 |

The inventors found similar correlations between the number of HUVECs sorted and the number of HUVECs recovered with flow cytometry ($R^2$=0.9991) and with RARE framework ($R^2$=0.9987) but all HUVECs were not detected. This may be due to the electronic abort of the cytometer and the sorting abort of the cell sorter.

To have clinical utility, the method in use must have a low variability and a validation of the true endothelial origin of cells designated as CECs by the assay in different pathologies (cancer and intracranial aneurysm treatment).

| | Counted CECs | |
|---|---|---|
| **Cancer** | **Kaluza™** | **RARE** |
| C1 | 2 | 7 |
| C2 | 15 | 25 |
| C3 | 38 | 41 |
| C4 | 35 | 32 |

| | Counted CECs | |
|---|---|---|
| **IA** | **Kaluza™** | **RARE** |
| A1 | 10 | 15 |
| A2 | 3 | 6 |
| A3 | 15 | 16 |
| A4 | 14 | 23 |
| A5 | 17 | 21 |
| A6 | 24 | 18 |

The CECs counted were not significantly different ($p > 0.05$) between analyzes on large datasets with a cytometry software (Kaluza™) and after the backward approach framework to isolate rare events in the same datasets.

References

1. Flow Through chamber for photometers to measure and particles in a dispersion medium. Dittrich and Göhde

2. Diagnostic and biological implications of flow cytometric DNA content analysis in lung cancer. Cancer Res., 1983, 43, 5026-5032. Bunn P & Al

3. Localization of antigen in tissue cells. II. Improvements in a method for the detection of antigens by means of fluorescent antibody. J. Exp. Med. 1950, 91, 1-10. Coons A.H. & Al

4. Multivariate chromosome analysis and complete karyotyping using dual labeling and fluorescence digital imaging microscopy. Cytometry, 1990, 11, 80-93. Arndt-Jovin D.J. & Al.

5. Image cytometric DNA analysis in human breast cancer analysis may add prognostic information in diploid cases with low S-phase fraction by flow cytometry. Cytometry, 1992, 13, 577-585. Baldetorp & Al

6. Single- and Double-stranded RNA measurements by flow cytometry in solid neoplasms. Cytometry, 1991, 12, 330-335. El-Naggar A.K. & Al

7. Flow cytometry measurement of cytoplasmic pH: a critical evaluation of available fluorochromes. Cytometry, 1986, 7, 347-355. Musgrove E. & Al

8. Analysis of cytosolic ionized calcium variation in polymorphonuclear leukocytes using flow cytometry and Indo-1 AM. Cytometry, 1989, 10, 165-173. Lopez M. & Al

**Claims**

1. Method for identifying a subpopulation of specific cells among a large population of cells, said method comprising the following steps:

   a. exposing the cells of said large population to reagents, said n reagents allowing the detection of the presence, of the absence or of the amount of n different components of each cell of said large population, n being greater than or equal to 2,

   b. detecting said reagents for each cells belonging to said large population, in order to assign each cell to a specific position within an n-dimensional space, and the following steps carried out by a computer program run on a computer

   c. grouping the cells by clusterisation into k different clusters, each of the clusters being **characterized by** a center and a radius, the clusterisation being such that from 80% to 90% of the cells belonging to said large population are assigned to one of said k clusters, wherein the clustering step is achieved by carrying out a modified k-means algorithm which is such that in the re-assignment phase of k-means only points that lie within a maximum radius DMAX around cluster centres are used to re-compute said cluster centres, so that only points that lie in dense regions are clustered by said modified k-means algorithm, and wherein the initial number of clusters KI and the maximum radius DMAX are parameters of the modified k-means algorithm selected so that the percentage of the cells belonging to said large population which are assigned to one of said k clusters lies in said range from 80% to 90%,

   d. grouping adjacent clusters to obtain larger clusters, two clusters being adjacent if the Euclidian distance between the centers of said two clusters is less than twice the maximum radius DMAX, and estimating the centers $C_{lk}$ of said larger clusters as well as the covariance matrix of the cells belonging to said larger cluster,

   e. defining sliding regions for each larger cluster by increasing the radius of said larger cluster by a factor $\varepsilon$, $\varepsilon$ varying from 0.01 to 0.1, and calculating the Mahalanobis distance for each cell that belongs to said sliding region,

   f. estimating the number of cells having a Mahalanobis distance lower than $D_{lk}(1+\varepsilon)$, but not belonging to the larger cluster, to measure and the density of said set, such that, if the density is greater than a value N, N being greater than 10, preferably N varies from 10 to 1000, in particular N varies from 10 to 500, then said set is considered to not contain said subpopulation of specific cells but to belong to the larger cluster, and steps e and f are repeated p times until the density of said set is lower than said value N, said set being defined by cells having a Mahalanobis distance lower than $D_{lk}(1+\varepsilon)^p$,

   g. identifying said subpopulation of specific cells as the set of cells which neither belong to a larger cluster nor to any considered sliding region.

2. Method according to claim 1, wherein said n- reagents are fluorescent reagents that interact with cellular proteins, lipids, glucids or nucleic acid molecules.

3. Method according to claim 1 or 2, wherein the detecting step b. is carried out by flow cytometry.

4. Method according to anyone of claims 1 to 3, wherein said large population of cells form all animal or human fluids such as blood sample, cerebrospinal fluid, amniotic fluid, bronchoalveolar lavage fluid, breast milk, cervicovaginal liquids.

5. Method according to anyone of claims 1 to 4, for identifying a subpopulation of mature endothelial cells in a blood sample, wherein the cells of the large population are labeled with at least the following markers: CD45, CD105 and CD146.

6. Method according to anyone of claims 1 to 4, for identifying a subpopulation of progenitor endothelial cells in a blood sample, wherein the cells of the large population are labeled with at least the following markers: CD45, CD34, CD133, and CD309.

7. Method according to anyone of claims 1 to 4, for identifying a subpopulation of epithelial cells, wherein the cells of the large population are labeled with at least the following markers: CD326, CD45, antibodies directed to cytokeratins.

8. Method according to anyone of claims 1 to 4, for identifying a subpopulation of regulating B cells or of Epstein-Barr Virus (EBV) infected memory B cells, wherein the cells of the large population are labeled with at least the following markers: CD27, CD24, CD19, and IL-10 or the following markers: CD27, CD19 and antibodies directed to EBV antigens, respectively.

9. Method according to anyone of claims 1 to 4, for identifying a subpopulation of regulating T cells, wherein the cells of the large population are labeled with at least the following markers: CD4, CD25, Foxp3 and antibodies directed to cytokines.

10. Method according to anyone of claims 1 to 4, for identifying a subpopulation of Human Immunodeficieny virus (HIV) infected CD4+ T cells, wherein the cells of the large population are labeled with at least the following markers: CD4, CD3, CD25 and antibodies directed to HIV antigens.

11. Method according to anyone of claims 1 to 10, wherein $\varepsilon = 10^{-1}$ and N= 10.

12. Method for the in vitro diagnosis or the in vitro prognosis of pathologies, including cancers, vascular and immune pathologies, and infectious diseases, said method comprising the step of identifying a subpopulation of specific cells among a large population of cells, as defined in anyone of claims 1 to 11.

13. Computer program on an appropriated support for identifying a subpopulation of specific cells among a large population of cells, wherein said large population of cells is subject to steps a to b according to anyone of claims 1-12, the computer program comprising means for causing a computer to carry out steps c. to g. of the method according to anyone of claim 1 to 12.

14. Kit comprising;

a. n- reagents, said n- reagents to detect of the presence, the absence or the amount of n- different components of each cells of said large population, n being upper than or equal to 2,
b. means for the detection of said n- different markers of cells, and
c. a computer program as defined in claim 13.

**Patentansprüche**

1. Verfahren zur Identifikation einer Teilpopulation spezifischer Zellen in einer großen Population an Zellen, wobei das Verfahren die folgenden Schritte umfasst:

a. Aussetzen der Zellen der großen Population gegenüber n Reagenzien, wobei die n Reagenzien die Detektion des Vorhandenseins, des Nichtvorhandenseins oder der Summe n verschiedener Komponenten jeder Zelle der großen Population ermöglichen, wobei n größer als oder gleich 2 ist,
b. Detektieren der n Reagenzien für jede Zelle, die zu der großen Population gehört, um jede Zelle einer spezifischen Position in einem n-dimensionalen Raum zuzuweisen,
und die folgenden Schritte, die durch ein auf einem Computer ausgeführtes Computerprogramm ausgeführt werden:

c. Gruppieren der Zellen durch Clusterbildung in k verschiedene Cluster, wobei jeder der Cluster durch einen Mittelpunkt und einen Radius gekennzeichnet ist, wobei die Clusterbildung derart ist, dass 80 % bis 90 % der Zellen, die zu der großen Population gehören, einem der k Cluster zugewiesen werden, wobei der Clusterbildungsschritt erreicht wird, indem ein modifizierter k-Means-Algorithmus ausgeführt wird, der derart ist, dass in der Neuzuweisungsphase von k-Means lediglich Punkte, die innerhalb eines maximalen Radius DMAX um Cluster-Mittelpunkte liegen, verwendet werden, um die Cluster-Mittelpunkte neu zu berechnen, sodass lediglich Punkte, die in dichten Regionen liegen, durch den modifizierten k-Means-Algorithmus geclustert werden, und wobei die anfängliche Anzahl an Clustern KI und der maximale Radius DMAX Parameter des modifizierten k-Means-Algorithmus sind, die derart ausgewählt sind, dass der Prozentsatz der Zellen, die zu der großen Population gehören, die einem der k Cluster zugewiesen werden, in der Spanne von 80 % bis 90 % liegt,

d. Gruppieren benachbarter Cluster, um größere Cluster zu erhalten, wobei zwei Cluster benachbart sind, wenn die euklidische Distanz zwischen den Mittelpunkten C der zwei Cluster weniger als das Doppelte des maximalen Radius DMAX beträgt,
und Schätzen der Mittelpunkte $C_{lk}$ der größeren Cluster sowie der Kovarianzmatrix der Zellen, die zu dem größeren Cluster gehören,

e. Definieren von Gleitregionen für jeden größeren Cluster durch das Erhöhen des Radius des größeren Clusters um einen Faktor $\varepsilon$, wobei $\varepsilon$ von 0,01 bis 0,1 variiert, und Berechnen der Mahalanobis-Distanz für jede Zelle, die zu der Gleitregion gehört,

f. Schätzen der Anzahl an Zellen, die eine Mahalanobis-Distanz von weniger als $D_{lk}(1+\varepsilon)$ aufweisen, aber nicht zu dem größeren Cluster gehören, um die Dichte des Satzes zu messen, sodass, wenn die Dichte größer als ein Wert N ist, wobei N größer als 10 ist, N bevorzugt von 10 bis 1000 variiert, N insbesondere von 10 bis 500 variiert, der Satz dann als die Teilpopulation spezifischer Zellen nicht enthaltend, aber zu dem größeren Cluster gehörend angesehen wird, und die Schritte e und f p-fach wiederholt werden, bis die Dichte des Satzes niedriger als der Wert N ist, wobei der Satz durch Zellen definiert ist, die eine Mahalanobis-Distanz aufweisen, die niedriger als $D_{lk}(1+\varepsilon)^p$ ist,

g. Identifizieren der Teilpopulation spezifischer Zellen als den Satz an Zellen, der weder zu einem größeren Cluster noch zu einer in Betracht gezogenen Gleitregion gehört.

2. Verfahren nach Anspruch 1, wobei die n Reagenzien fluoreszierende Reagenzien sind, die mit zellulären Proteinen, Lipiden, Kohlenhydraten oder Nukleinsäuremolekülen interagieren.

3. Verfahren nach Anspruch 1 oder 2, wobei der Detektionsschritt b. durch Durchflusszytometrie ausgeführt wird.

4. Verfahren nach einem der Ansprüche 1 bis 3, wobei die große Population an Zellen alle tierischen oder menschlichen Fluide wie zum Beispiel Blutprobe, Liquor, amniotisches Fluid, bronchoalveoläres Lavage-Fluid, Muttermilch, zervikovaginale Flüssigkeiten bilden.

5. Verfahren nach einem der Ansprüche 1 bis 4 zur Identifikation einer Teilpopulation maturer Endothelzellen in einer Blutprobe, wobei die Zellen der großen Population mit zumindest den folgenden Markern markiert sind: CD45, CD105 und CD146.

6. Verfahren nach einem der Ansprüche 1 bis 4 zur Identifikation einer Teilpopulation an Vorläuferendothelzellen in einer Blutprobe, wobei die Zellen der großen Population mit zumindest den folgenden Markern markiert sind: CD45, CD34, CD133 und CD309.

7. Verfahren nach einem der Ansprüche 1 bis 4 zur Identifikation einer Teilpopulation an Epithelzellen, wobei die Zellen der großen Population mit zumindest den folgenden Markern markiert sind: CD326, CD45, Antikörper gegen Zytokeratine.

8. Verfahren nach einem der Ansprüche 1 bis 4 zur Identifikation einer Teilpopulation regulatorischer B-Zellen oder Epstein-Barr-Virus (EBV)-infizierter B-Gedächtniszellen, wobei die Zellen der großen Population mit zumindest den folgenden Markern markiert sind: CD27, CD24, CD19 und IL-10, oder den folgenden Markern: jeweils CD27, CD19 und Antikörper gegen EBV-Antigene.

9. Verfahren nach einem der Ansprüche 1 bis 4 zur Identifikation einer Teilpopulation regulatorischer T-Zellen, wobei die Zellen der großen Population mit zumindest den folgenden Markern markiert sind: CD4, CD25, Foxp3 und Antikörper gegen Zytokine.

**10.** Verfahren nach einem der Ansprüche 1 bis 4 zur Identifikation einer Teilpopulation Humanes Immundefizienz-Virus (HIV)-infizierter CD4+-T-Zellen, wobei die Zellen der großen Population mit zumindest den folgenden Markern markiert sind: CD4, CD3, CD25 und Antikörper gegen HIV-Antigene.

**11.** Verfahren nach einem der Ansprüche 1 bis 10, wobei $\varepsilon = 10^{-1}$ und N = 10.

**12.** Verfahren für die In-vitro-Diagnose oder die In-vitro-Prognose von Pathologien, darunter Krebs, vaskuläre und immunologische Pathologien, und infektiösen Erkrankungen, wobei das Verfahren den Schritt der Identifikation einer Teilpopulation spezifischer Zellen in einer großen Population an Zellen nach einem der Ansprüche 1 bis 11 umfasst.

**13.** Computerprogramm auf einem geeigneten Träger zur Identifikation einer Teilpopulation spezifischer Zellen in einer großen Population an Zellen, wobei die große Population an Zellen den Schritten a. bis b. nach einem der Ansprüche 1-12 unterzogen wird, wobei das Computerprogramm Mittel umfasst, um zu bewirken, dass ein Computer die Schritte c. bis g. des Verfahrens nach einem der Ansprüche 1 bis 12 ausführt.

**14.** Kit, umfassend:

a. n Reagenzien, wobei die n Reagenzien zum Detektieren des Vorhandenseins, des Nichtvorhandenseins oder der Summe n verschiedener Komponenten jeder Zelle der großen Population dienen, wobei n größer als oder gleich 2 ist,
b. Mittel zur Detektion der n verschiedenen Marker von Zellen, und
c. ein Computerprogramm nach Anspruch 13.

## Revendications

**1.** Procédé d'identification d'une sous-population de cellules spécifiques parmi une grande population de cellules, ledit procédé comprenant les étapes suivantes :

a. exposer les cellules de ladite grande population à n réactifs, lesdits n réactifs permettant la détection de la présence, de l'absence ou de la quantité de n composants différents de chaque cellule de ladite grande population, n étant supérieur ou égal à 2,
b. détecter lesdits n réactifs pour chaque cellule appartenant à ladite grande population, afin d'affecter à chaque cellule une position spécifique dans un espace à n dimensions,
et les étapes suivantes effectuées par un programme informatique sur un ordinateur
c. regrouper les cellules par partitionnement en k clusters différents, chacun des clusters étant **caractérisé par** un centre et un rayon, le partitionnement étant tel que de 80 % à 90 % des cellules appartenant à ladite grande population sont affectées à l'un desdits k clusters, l'étape de partitionnement étant réalisée par exécution d'un algorithme des k-moyennes modifié qui est tel que, dans la phase de réaffectation des k-moyennes, seuls les points qui se trouvent dans un rayon maximum DMAX autour des centres de cluster sont utilisés pour recalculer lesdits centres de cluster, de sorte que seuls les points qui se trouvent dans des régions denses sont partitionnés par ledit algorithme des k-moyennes modifié, et le nombre initial de clusters KI et le rayon maximal DMAX étant des paramètres de l'algorithme des k-moyennes modifié sélectionné de sorte que le pourcentage des cellules appartenant à ladite grande population qui sont affectées à l'un desdits k clusters se trouve dans ladite gamme de 80 % à 90 %,
d. regrouper des clusters adjacents pour obtenir des clusters plus grands, deux clusters étant adjacents si la distance euclidienne entre les centres C desdits deux clusters est inférieure à deux fois le rayon maximal DMAX, et estimer les centres $C_{lk}$ desdits clusters plus grands ainsi que la matrice de covariance des cellules appartenant audit cluster plus grand,
e. définir des régions glissantes pour chaque cluster plus grand par augmentation du rayon dudit cluster plus grand d'un facteur $\varepsilon$, $\varepsilon$ variant de 0,01 à 0,1, et calculer la distance de Mahalanobis pour chaque cellule appartenant à ladite région glissante,
f. estimer le nombre de cellules ayant une distance de Mahalanobis inférieure à $D_{lk}(1+\varepsilon)$, mais n'appartenant pas au cluster plus grand, pour mesurer la densité dudit ensemble, de sorte que, si la densité est supérieure à une valeur N, N étant supérieur à 10, de préférence N variant de 10 à 1 000, en particulier N variant de 10 à 500, alors ledit ensemble est considéré comme ne contenant pas ladite sous-population de cellules spécifiques mais comme appartenant au cluster plus grand,
et les étapes e et f sont répétées p fois jusqu'à ce que la densité dudit ensemble soit inférieure à ladite valeur N, ledit ensemble étant défini par des cellules ayant une distance de Mahalanobis inférieure à $D_{lk}(1+\varepsilon)^p$,
g. identifier ladite sous-population de cellules spécifiques comme étant l'ensemble des cellules qui n'appartiennent ni à un cluster plus grand ni à une quelconque région glissante considérée.

2. Procédé selon la revendication 1, dans lequel lesdits n réactifs sont des réactifs fluorescents qui interagissent avec les protéines cellulaires, les lipides, les glucides ou les molécules d'acide nucléique.

3. Procédé selon la revendication 1 ou 2, dans lequel l'étape de détection b. est réalisée par cytométrie en flux.

4. Procédé selon l'une quelconque des revendications 1 à 3, dans lequel ladite grande population de cellules forme tous les fluides animaux ou humains tels qu'un échantillon de sang, le liquide céphalo-rachidien, le liquide amniotique, le liquide de lavage broncho-alvéolaire, le lait maternel, les liquides cervico-vaginaux.

5. Procédé selon l'une quelconque des revendications 1 à 4, d'identification d'une sous-population de cellules endothéliales matures dans un échantillon de sang, les cellules de la grande population étant marquées avec au moins les marqueurs suivants : CD45, CD105 et CD146.

6. Procédé selon l'une quelconque des revendications 1 à 4, d'identification d'une sous-population de cellules endothéliales progénitrices dans un échantillon de sang, les cellules de la grande population étant marquées avec au moins les marqueurs suivants : CD45, CD34, CD133 et CD309.

7. Procédé selon l'une quelconque des revendications 1 à 4, d'identification d'une sous-population de cellules épithéliales, les cellules de la grande population étant marquées avec au moins les marqueurs suivants : CD326, CD45, les anticorps dirigés contre les cytokératines.

8. Procédé selon l'une quelconque des revendications 1 à 4, d'identification d'une sous-population de lymphocytes B régulateurs ou les lymphocytes B à mémoire infectés par le virus Epstein-Barr (EBV), les cellules de la grande population étant marquées avec au moins les marqueurs suivants : CD27, CD24, CD19 et IL-10 ou les marqueurs suivants : CD27, CD19 et les anticorps dirigés contre les antigènes EBV, respectivement.

9. Procédé selon l'une quelconque des revendications 1 à 4, d'identification d'une sous-population de lymphocytes T régulateurs, les cellules de la grande population étant marquées avec au moins les marqueurs suivants : CD4, CD25, Foxp3 et les anticorps dirigés contre les cytokines.

10. Procédé selon l'une quelconque des revendications 1 à 4, d'identification d'une sous-population de lymphocytes T CD4+ infectées par le virus de l'immunodéficience humaine (VIH), les cellules de la grande population étant marquées avec au moins les marqueurs suivants : CD4, CD3, CD25 et les anticorps dirigés contre les antigènes du VIH.

11. Procédé selon l'une quelconque des revendications 1 à 10, dans lequel $\varepsilon = 10^{-1}$ et N = 10.

12. Procédé de diagnostic in vitro ou de pronostic in vitro de pathologies, incluant les cancers, les pathologies vasculaires et immunitaires, et les maladies infectieuses, ledit procédé comprenant l'étape d'identification d'une sous-population de cellules spécifiques parmi une grande population de cellules, telle que définie dans l'une quelconque des revendications 1 à 11.

13. Programme informatique sur un support approprié destiné à identifier une sous-population de cellules spécifiques parmi une grande population de cellules, ladite grande population de cellules étant soumise aux étapes a à b selon l'une quelconque des revendications 1 à 12, le programme informatique comprenant des moyens pour qu'un ordinateur effectue les étapes c. à g. du procédé selon l'une quelconque des revendications 1 à 12.

14. Kit comprenant :

a. n réactifs, lesdits n réactifs étant destinés à détecter la présence, l'absence ou la quantité de n composants différents de chaque cellule de ladite grande population, n étant supérieur ou égal à 2,

b. des moyens de détection desdits n marqueurs différents de cellules, et
c. un programme informatique tel que défini dans la revendication 13.

Fig. 1A

Fig. 1B

**Figures 1**

Fig. 2A

Fig. 2B

Fig. 2C

**Figures 2**

Fig. 3A | Fig. 3B | Fig. 3C
Fig. 3D | Fig. 3E | Fig. 3F
Fig. 3G | Fig. 3H | Fig. 3I

**Figures 3**

Fig. 4A | Fig. 4B | Fig. 4C

**Figures 4**

**Figure 5**

(a) LOF (*MinPts* = 10, *Thresh* = 1.5)

(b) LOF (*MinPts* = 20, *Thresh* = 1.5)

(c) LOF (*MinPts* = 30, *Thresh* = 1.5)

(d) LOF (*MinPts* = 50, *Thresh* = 1.5)

(e) LOF (*MinPts* = 50, *Thresh* = 2)

(f) RARE ($k = 4$, $D_{MAX} = 2$, $N_S = 10$)

(g) RARE ($k = 5$, $D_{MAX} = 2$, $N_S = 10$)

(h) RARE ($k = 7$, $D_{MAX} = 1.5$, $N_S = 10$)

(i) RARE ($k = 10$, $D_{MAX} = 1$, $N_S = 10$)

Figure 6

**Figure 7A**

Figure 7B

Figure 8

(a) LOCI Radius=3000      (b) LOCI Radius=4000

(c) LOCI Radius=5000      (d) LOCI Radius=6000

Figure 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006089190 A

**Non-patent literature cited in the description**

- **Y. GE et al.** flowPeaks: a fast unsupervised clustering for flow cytometry data via K-means and density peak finding. *BIOINFORMATICS,* August 2012, vol. 28 (15), 2052-2058
- **D.H. BAE ; S. JEONG ; S.W. KIM ; M. LEE.** Outlier detection using centrality and center-proximity. *In Proceedings of CIKM,* 2012
- **A. BASHASHATI ; R. BRINKMAN.** A survey of ow cytometry data analysis methods. *In Advances in Bioinformatics,* 2009
- **M. BREUNIG ; H.P. KRIEGEL ; R.T. NG ; J. SANDER.** LOF: identifying densitybased local outliers. *In Proceedings of ACM SIGMOD,* 2000
- **V. CHANDOLA ; A. BANERJEE ; V. KUMAR.** Anomaly detection: a survey. *ACM Computing Surveys,* 2009, vol. 41
- **L. ERTÖZ ; M. STEINBACH ; V. KUMAR.** Finding clusters of di_erent sizes, shapes and densities in noisy, high-dimensional data. *SDM,* 2003
- **M. ESTER ; H.P. KRIEGEL ; J. SANDER ; X. XU.** A density-based algorithm for discovering clusters in large spatial databases with noise. *Proceedings of ACM SIGKDD,* 1996
- **Z. HE ; X. XU ; S. DENG.** Discovering cluster-based local outliers. *Pattern Recognition Letters,* 2003, vol. 24
- **E. LEVINA ; P.J. BICKEL.** Maximum likelihood estimation of intrinsic dimension. *Advances in Neural Information Processing Systems,* 2005, vol. 17
- **U. VON LUXBURG.** A tutorial on spectral clustering. *Statistics and Computing,* 2007, vol. 17
- **J.B. MACQUEEN.** Some methods for classification and analysis of multivariate observations. *Proceedings of the Fifth Berkeley Symposium on Mathematical Statistics and Probability,* 1967
- **S. PAPADIMITRIOU ; H. KITAGAWA ; P. GRIBBONS ; C. FALOUTSOS.** LOCI: Fast outlier detection using the local correlation integral. *Proceedings of ICDE,* 2003

- **Y. TANG ; Y. Q. ZHANG ; N. W. CHAWLA ; S. KRASSER.** SVMs for highly imbalanced classification. *IEEE Transactions on Systems, Man and Cybernetics,* 2009, vol. 39, 281-288
- **H. XIONG ; J. WU ; J. CHEN.** K-means clustering versus validation measures: a data distribution perspective. *KDD,* 2006
- Data clustering with size constraints. **S. ZHU ; D. WANG ; T. LI.** Knowledge-Based Systems. Elsevier, 2010, vol. 23, 883-889
- **BUNN P.** Diagnostic and biological implications of flow cytometric DNA content analysis in lung cancer. *Cancer Res.,* 1983, vol. 43, 5026-5032
- **COONS A.H.** Localization of antigen in tissue cells. II. Improvements in a method for the detection of antigens by means of fluorescent antibody. *J. Exp. Med.,* 1950, vol. 91, 1-10
- **ARNDT-JOVIN D.J.** Multivariate chromosome analysis and complete karyotyping using dual labeling and fluorescence digital imaging microscopy. *Cytometry,* 1990, vol. 11, 80-93
- **BALDETORP.** Image cytometric DNA analysis in human breast cancer analysis may add prognostic information in diploid cases with low S-phase fraction by flow cytometry. *Cytometry,* 1992, vol. 13, 577-585
- **EL-NAGGAR A.K.** Single- and Double-stranded RNA measurements by flow cytometry in solid neoplasms. *Cytometry,* 1991, vol. 12, 330-335
- **MUSGROVE E.** Flow cytometry measurement of cytoplasmic pH: a critical evaluation of available fluorochromes. *Cytometry,* 1986, vol. 7, 347-355
- **LOPEZ M.** Analysis of cytosolic ionized calcium variation in polymorphonuclear leukocytes using flow cytometry and Indo-1 AM. *Cytometry,* 1989, vol. 10, 165-173